# EUROPEAN PATENT APPLICATION

(11) **EP 4 670 741 A1**
(43) Date of publication of application: **31.12.2025**
(21) Application number: 24797502.2
(22) Date of filing: 29.04.2024
(51) Int. Cl.: A61K 47/68

(54) **ANTIBODY-DRUG CONJUGATE IN WHICH ONE OR MORE DRUG-LINKER CONJUGATES ARE LINKED TO ANTIBODY AND METHOD FOR PREPARING SAME**

(30) Priority: 28.04.2023 KR 20230055967
(71) Applicant: Pinotbio, Inc., Gyeonggi-do 16506 (KR)
(72) Inventor: JUNG, Doo Young, Suwon-si, Gyeonggi-do 16506 (KR); LEE, Byeong Sung, Suwon-si, Gyeonggi-do 16506 (KR)
(74) Representative: Prüfer & Partner mbB Patentanwälte · Rechtsanwälte
(86) International application number: PCT/KR2024/005811
(87) International publication number: WO 2024/225848

(57) **Abstract**

The present invention relates to an antibody-drug conjugate (ADC) in which one or more drug-linker conjugates are linked to an antibody and an efficient method for preparing same. Particularly, each of at least one type of drug-linker conjugate selected from the group consisting of: a drug-linker conjugate (A) which is a combination of a camptothecin-based drug and an acid-sensitive linker; a drug-linker conjugate (B) which is a combination of a non-camptothecin-based super-toxin drug and an enzyme-sensitive linker; a drug-linker conjugate (C) which is a combination of a camptothecin-based drug and an enzyme-sensitive linker; and a drug-linker conjugate (D) which is a combination of an antiapoptotic protein inhibitor drug and an enzyme-sensitive linker may be linked to cysteine and lysine residues of an antibody.

## Description

### Technical Field

The present invention relates to an antibody-drug conjugate (ADC) in which one or more drug-linker conjugates are bound conjugated to an antibody, and an efficient method for preparing same.

Specifically, wherein the one or more drug-linker conjugates are selected from the group consisting of: a drug-linker conjugate(A) consisting of the combination of a camptothecin-based drug and an acid-sensitive linker; a drug-linker conjugate(B) consisting of the combination of a non-camptothecin-based super-toxic drug and an enzyme-sensitive linker; a drug-linker conjugate(C) consisting of the combination of a camptothecin-based drug and an enzyme-sensitive linker; and a drug-linker conjugate(D) consisting of the combination of an anti-apoptotic protein inhibitor drug and an enzyme-sensitive linker, and wherein each of the one or more drug-linker conjugates is conjugated to an amino acid residue of the antibody.

### Related Art

An antibody-drug conjugate (ADC) is a novel drug platform that selectively delivers a potent cytotoxic payload specifically to tumor tissues by utilizing the high tissue specificity of antibodies. ADCs are capable of selectively delivering potent payloads that induce cancer cell death even at picomolar concentrations to tumor tissues, while minimizing systemic drug exposure, thereby achieving both antitumor efficacy and safety.

The ADC technology delivers drugs to tumor cells by using antibodies that specifically bind to antigens expressed on the surface of cancer cells. The cellular internalization of most ADCs proceeds through clathrin-coated pit mechanisms. After internalization, the ADC dissociates from the clathrin and fuses with other intracellular vesicles, proceeding through the endosome-lysosome pathway. Subsequently, proteases in the acidic environment of the endosome cleave the linker, and the activated "free" drug crosses the lysosomal membrane and enters the cytoplasm, where it binds to its molecular target. This binding halts the cell cycle of the tumor cell and leads to apoptosis, thereby causing cancer cell death. A portion of the drug may passively diffuse, be actively transported, or leak out through dead cells. If the leaked drug is membrane-permeable, it may enter neighboring cells, resulting in a so-called "bystander cell-killing" effect.

An ADC is primarily composed of three components:
(i) An antibody that specifically targets an antigen selectively expressed on tumor cells over normal cells;
(ii) A cytotoxic payload designed to kill the target cancer cells; and
(iii) A chemical linker that covalently connects the payload to the antibody.

In other words, an ADC is an antibody-based therapeutic agent that combines the targeting ability of a monoclonal antibody with the potent cytotoxicity of a cytotoxic payload.

The potency of the cytotoxic payload conjugated to an ADC is typically 100 to 1,000 times greater than that of the free drug alone. The goal of ADC development is to produce drugs that exhibit high specificity toward target cancer cells while minimizing severe adverse effects on normal tissues.

To develop ADCs, it is essential to understand the selection of target antigens, the internalization of ADCs by tumor cells, the potency of the drug, and the stability of the linker between the drug and the antibody. Furthermore, the conjugation method between the cytotoxic payload and the antibody, the drug-to-antibody ratio (DAR), and the effect of the linker type and antibody characteristics on drug attachment efficiency are crucial for developing safe and effective ADCs.

Challenges in ADC development include competition with existing therapeutics and other innovative antibody drugs, relatively complex manufacturing processes compared to conventional therapies, high production costs, instability of the linker, and heterogeneous DAR profiles.

In particular, the stability of the linker connecting the antibody and cytotoxic payload is closely related to toxicity in clinical trials.

A major issue in ADCs is the premature cleavage of the linker in normal tissues rather than in the target cancer cells, which may result in unintended toxicity. The first ADC approved in the U.S. market, Mylotarg, was withdrawn in 2010 due to the instability of its antibody-conjugated linker and the resulting adverse effects caused by the potent toxicity of the drug. Additional toxicity issues may arise when ADCs target antigens that are also expressed in normal tissues.

ADCs that have been approved to date, as well as those in the clinical pipeline, are manufactured by conjugating low-molecular-weight cytotoxic payloads to lysine or cysteine residues of antibodies via linkers. For instance, Adcetris, Kadcyla, and the re-approved Mylotarg (2017) are produced through processes in which the number of drug molecules attached to the antibody is only partially controlled. According to studies on the DAR profile of Kadcyla, an average of four drugs are bound per antibody, but since monoclonal antibodies possess approximately 80 lysine residues, and 8-10 surface-exposed lysines are highly reactive, the DAR can vary widely from 0 to 8. Companies including Pfizer (e.g., Besponsa/Inotuzumab ozogamicin, approved in 2017) are developing platforms to achieve a more defined DAR.

However, from the early stages of development, heterogeneity in ADCs like T-DM1 (trade name: Kadcyla^{®}) has been problematic. Random conjugation to approximately 70-80 lysine residues of the antibody leads to inconsistency in the DAR and conjugation sites. Conventional random conjugation results in a DAR range of 0-8 and multiple ADC species with varying drug loads. Recent reports indicate that changes in drug loading and conjugation sites alter pharmacokinetics, drug release rates, and therapeutic efficacy, necessitating precise control over the number and site of drug conjugation in next-generation ADCs. Precise control over the number and location of drug molecules conjugated to the antibody could improve expected efficacy, reduce variation in conjugates and manufacturing lots, and address regulatory challenges.

Site-specific modification methods include genetic engineering or enzyme-based approaches. Genetic engineering enables control over both site and number of modifications.

Recently, a method called CCAP (Chemical Conjugation by Affinity Peptide) has been developed (US 10227383 B2; US 2021/0139548 A1; ACS Omega (2019) Vol. 4, pp. 20564-20570; all incorporated herein by reference). CCAP method achieves site-selective conjugation of antibodies via reacting a peptide reagent-comprising a drug and an NHS-activated ester covalently bound to an affinity peptide-with the antibody(That is, an ADC preparing method employing a linker containing a peptide moiety). Notably, the CCAP method represents the first chemical synthesis-based technique to achieve site-specific modification of the Fc domain of antibodies with cytotoxic drugs. This approach demonstrates highly practical utility, with a short reaction time (~30 minutes), high yield (~70% for DAR 1), and complete site selectivity (100%). It has been demonstrated that the addition of approximately five molar equivalents of the peptide reagent allows for the control of the DAR to 2. Moreover, the ability to precisely control the conjugation site renders this method highly innovative.

One of the most critical factors influencing the pharmacokinetic properties and in vivo distribution of an ADC is the drug-to-antibody ratio (DAR). ADCs with higher DARs tend to exhibit greater potency in in vitro experiments. However, such ADCs often show reduced efficacy in vivo, which is believed to be due to their increased plasma clearance compared to ADCs with lower DARs. As a result of these observations, most currently approved and developing ADCs are engineered to maintain a DAR in the range of 2 to 4. Accordingly, drug conjugation technologies targeting cysteine or lysine residues of antibodies are commonly used. While many attempts have been made to increase DAR, most have failed. The main reason for this failure is the hydrophobic nature of both the cytotoxic payloads and linkers. Additional contributing factors include aggregation of the ADC, loss of affinity for the target antigen, or increased plasma clearance. Recently, a homogeneous ADC with a DAR of 8 was developed using cysteine conjugation. However, even this ADC exhibited increased plasma clearance, which is attributed to the unique structure of the drug-linker complex and the extensive modifications made to the antibody.

The concept of an "ideal" ADC is to deliver the maximum amount of cytotoxic payload specifically to target cancer cells. To develop high-DAR ADCs that can persist in the plasma for extended periods, it is necessary to finely balance between the number of drug molecules conjugated to the antibody and the degree of antibody modification. In this regard, further technological development is required, including the use of hydrophilic linkers such as PEG-based linkers or branched linkers to overcome the limitations of conventional ADC linker systems.

As the ADC field evolves, the goal is no longer merely to selectively deliver potent chemotherapeutic agents or radionuclei to tumors via antibodies, but to efficiently deliver payloads with diverse pharmacological activities, or combinations thereof, to tumor tissues in order to maximize anticancer efficacy. Although highly potent payloads such as MMAE, Hemiasterlin, and PBD have been incorporated into ADCs, their clinical efficacy in humans has often fallen short of expectations based on preclinical animal studies. To improve the efficacy of ADCs, strategies involving combination payloads are being explored. For example, combinations of anti-apoptotic protein inhibitors (such as Bcl-XL inhibitors like Navitoclax) with MMAE or Topoisomerase I inhibitors, and combinations of CHEK1 inhibitors with Topoisomerase I inhibitors have been studied. However, efficient preparing of dual-payload ADCs remains technically challenging. In current clinical strategies, highly potent drugs such as MMAE or Topoisomerase I inhibitors are typically used in the form of ADCs, while combination agents such as anti-apoptotic protein inhibitors are administered systemically. For instance, AbbVie has developed an ADC named ABBV-155 with a Bcl-XL inhibitor as the payload. However, there is growing concern that the efficiency of drug delivery may be limited when two different ADCs are used, due to the limited number of antigens expressed on the surface of cancer cells. Moreover, compared to conventional chemotherapeutic agents, the high cost of ADCs poses a significant barrier to the clinical application of combination therapies involving two or more ADCs. Debiopharm has developed a linker-payload system in which two drugs are conjugated at a fixed 1:1 ratio, but this system still faces limitations, such as relatively high aggregation rates and the inability to vary the payload ratio. Therefore, a new ADC structure that can efficiently deliver one or more payloads in flexible ratios while minimizing the risk of aggregation has yet to be realized in practice.

### SUMMARY OF THE INVENTION

### TECHNICAL PROBLEM

The present invention has been devised to address the aforementioned problems, provides an antibody-drug conjugate (ADC) in which one or more drug-linker conjugates are conjugated to a single antibody via linkers, thereby enabling control of the pharmacokinetic properties and in vivo distribution as desired.

### TECHNICAL SOLUTION

A first aspect of the present invention provides an antibody-drug conjugate (ADC), in which one or more drug-linker conjugates are conjugated to an antibody, wherein the one or more drug-linker conjugates are selected from the group consisting of: a drug-linker conjugate(A) consisting of the combination of a camptothecin-based drug and an acid-sensitive linker; a drug-linker conjugate(B) consisting of the combination of a non-camptothecin-based super-toxic drug and an enzyme-sensitive linker; a drug-linker conjugate(C) consisting of the combination of a camptothecin-based drug and an enzyme-sensitive linker; and a drug-linker conjugate(D) consisting of the combination of an anti-apoptotic protein inhibitor drug and an enzyme-sensitive linker, and wherein each of the one or more drug-linker conjugates is conjugated to a cysteine or lysine residue of the antibody.

According to the first aspect of the present invention, a second aspect provides a method for preparing an antibody-drug conjugate(ADC) in which one or more drug-linker conjugates are conjugated to a single antibody, comprising: selecting one or more drug-linker conjugates from the group consisting of: a drug-linker conjugate(A) consisting of the combination of a camptothecin-based drug and an acid-sensitive linker; a drug-linker conjugate(B) consisting of the combination of a non-camptothecin-based super-toxic drug and an enzyme-sensitive linker; a drug-linker conjugate(C) consisting of the combination of a camptothecin-based drug and an enzyme-sensitive linker; and a drug-linker conjugate(D) consisting of the combination of an anti-apoptotic protein inhibitor drug and an enzyme-sensitive linker; and conjugating each drug-linker conjugate to a cysteine or lysine residue of the antibody by employing, depending on the drug-linker conjugate, different (i) amino acid residues at the binding site on the antibody, (ii) conjugation sequences, and/or (iii) conjugation methods.

Hereinafter, the present invention will be described in detail.

In the specification, the term "antibody" refers to a protein molecule that acts as a ligand that specifically recognizes an antigen, including an immunoglobulin molecule that is immunologically reactive with a specific antigen, and includes all of polyclonal antibody, a monoclonal antibody, and a whole antibody. The above term also includes a chimeric antibody, a bivalent or bispecific molecule, a diabody, a triabody, and a tetrabody. The antibody further includes single chain antibodies (scABs) with a binding function to FcRn, derivatives of antibody constant regions, and artificial antibodies based on protein scaffolds. A whole antibody is a structure having two full-length light chains and two full-length heavy chains, wherein each light chain linked to a heavy chain by a disulfide bond. The whole antibodies include IgA, IgD, IgE, IgM, and IgG, wherein IgG is a subtype, including IgG1, IgG2, IgG3, and IgG4.

Preferably, the antibody is at least one selected from the group consisting of Urelumab, Utomilumab, Bebtelovimab, Aducanumab, Bapinezumab, Crenezumab, Donanemab, Gantenerumab, Lecanemab, Solanezumab, Nesvacumab, Evinacumab, Enoblituzumab, Omburtamab, Belimumab, Ianalumab, Tabalumab, Bertilimumab, Mogamulizumab, Leronlimab, Siplizumab, Foralumab, Muromonab-CD3, Otelixizumab, Teplizumab, Ibalizumab, Tregalizumab, Zanolimumab, Itolizumab, Efalizumab, Inebilizumab, Tafasitamab, Tositumomab, Ocrelizumab, Ofatumumab, Rituximab, Ublituximab, Veltuzumab, Epratuzumab, Basiliximab, Daclizumab, Varlilumab, Lulizumab, Iratumumab, Lintuzumab, Daratumumab, Felzartamab, Isatuximab, Mezagitamab, Bleselumab, Dacetuzumab, Iscalimab, Lucatumumab, Mitazalimab, Sotigalimab, Dapirolizumab, Apamistamab, Ligufalimab, Magrolimab, Alemtuzumab, Crizanlizumab, Inclacumab, Cusatuzumab, Oleclumab, Milatuzumab, Galiximab, Carotuximab, Adecatumumab, Eptinezumab, Erenumab, Fremanezumab, Galcanezumab, Zolbetuximab, Onartuzumab, Eculizumab, Pozelimab, Ravulizumab, Lacnotuzumab, Axatilimab, Cabiralizumab, Emactuzumab, Ipilimumab, Quavonlimab, Tremelimumab, Zalifrelimab, Cetuximab, Depatuxizumab, Futuximab, Imgatuzumab, Matuzumab, Modotuximab, Necitumumab, Nimotuzumab, Panitumumab, Tomuzotuximab, Zalutumumab, Batoclimab, Nipocalimab, Rozanolixizumab, Burosumab, Farletuzumab, Dinutuximab, Naxitamab, Ragifilimab, Gimsilumab, Lenzilumab, Mavrilimumab, Namilumab, Otilimab, Plonmarlimab, Codrituzumab, Margetuximab, Pertuzumab, Trastuzumab, Datopotamab, Patritumab, Seribantumab, Duligotuzumab, Ficlatuzumab, Rilotumumab, Alomfilimab, Anifrolumab, Emapalumab, Ligelizumab, Omalizumab, Cixutumumab, Dalotuzumab, Figitumumab, Ganitumab, Teprotumumab, Bermekimab, Canakinumab, Gevokizumab, Briakinumab, Ustekinumab, Anrukinzumab, Cendakimab, Lebrikizumab, Tralokinumab, Brodalumab, Bimekizumab, Ixekizumab, Secukinumab, Brazikumab, Guselkumab, Mirikizumab, Risankizumab, Tildrakizumab, Nemolizumab, Imsidolimab, Spesolimab, Pascolizumab, Dupilumab, Depemokimab, Mepolizumab, Reslizumab, Benralizumab, Clazakizumab, Olokizumab, Siltuximab, Sirukumab, Ziltivekimab, Levilimab, Sarilumab, Satralizumab, Tocilizumab, Abituzumab, Favezelimab, Fianlimab, Ieramilimab, Relatlimab, Simtuzumab, Abagovomab, Oregovomab, Tanezumab, Ivuxolimab, Rocatinlimab, Tavolimab, Telazorlimab, Vonlerolizumab, Alirocumab, Bococizumab, Ebronucimab, Evolocumab, Frovocimab, Ongericimab, Tafolecimab, Dostarlimab, Balstilimab, Camrelizumab, Cemiplimab, Geptanolimab, Nivolumab, Pembrolizumab, Penpulimab, Pidilizumab, Prolgolimab, Retifanlimab, Sasanlimab, Serplulimab, Sintilimab, Spartalizumab, Tislelizumab, Toripalimab, Ezabenlimab, Zimberelimab, Atezolizumab, Avelumab, Cosibelimab, Sugemalimab, Durvalumab, Envafolimab, Suvratoxumab, Denosumab, Zilovertamab, Elotuzumab, Domvanalimab, Etigilimab, Ociperlimab, Tiragolumab, Vibostolimab, Surzebiclimab, Cobolimab, Sabatolimab, Concizumab, Marstacimab, Adalimumab, Golimumab, Infliximab, Certolizumab, Conatumumab, Tigatuzumab, Tezepelumab, Gatipotuzumab, Cabiralizumab, Bevacizumab, Brolucizumab, Ranibizumab, Olinvacimab, Icrucumab, Ramucirumab, Caplacizumab, Abrilumab, Etrolizumab, Vedolizumab, Intetumumab, Natalizumab, Obrindatamab, Elranatamab, Linvoseltamab, Teclistamab, Epcoritamab, Glofitamab, Mosunetuzumab, Odronextamab, Flotetuzumab, Vibecotamab, Catumaxomab, Cibisatamab, Talquetamab, Ubamatamab, Emfizatamab, Blinatumomab, Amivantamab, Emicizumab, Zenocutuzumab, Zanidatamab, Tibulizumab, Naptumomab, Belantamab, Pivekimab, Praluzatamab, Coltuximab, Denintuzumab, Loncastuximab, Ibritumomab, Inotuzumab, Epratuzumab, Moxetumomab, Brentuximab, Gemtuzumab, Vadastuximab, Lorvotuzumab, Polatuzumab, Tusamitamab, Telisotuzumab, Rovalpituzumab, Depatuxizumab, Farletuzumab, Mirvetuximab, Disitamab, Anetumab, Enfortumab, Sacituzumab, Vobarilizumab, Cadonilimab, Vudalimab, Tebotelimab, Ivonescimab, Erfonrilimab, Ozoralizumab, Faricimab, Vanucizumab, and Navicixizumab.

In the specification, the term "drug-linker conjugate" refers to a material for preparing an ADC, in which the antibody is not yet connected.

In the specification, the term "drug-linker conjugate being conjugated" refers to a conjugation state in which the drug-to-antibody ratio (DAR) and/or the conjugation site(s) on the antibody is uniformly defined.

Each of the one or more (preferably two) drug-linker conjugates of the present invention may be conjugated to an amino acid residue containing cysteine or lysine, a non-natural amino acid residue, or a glycan of the antibody.

Even when linkers are conjugated to the same amino acid sequence of an antibody, the steric or electromagnetic environment can vary depending on the type and length of the linker, and the conjugation position on the antibody, which in turn may influence in vivo biotransformation due to deconjugation of the linker. Additionally, the ADC should retain antigen-binding affinity equivalent to that of the unconjugated antibody. That is, the conjugation of drugs to the antibody should not interfere with antibody-antigen binding.

Therefore, in consideration of the above, various combinations of drug-linker conjugates can be rationally designed.

In the present invention, drug-linker conjugates are classified into: a drug-linker conjugate(A) consisting of the combination of a camptothecin-based drug and an acid-sensitive linker; a drug-linker conjugate(B) consisting of the combination of a non-camptothecin-based super-toxic drug and an enzyme-sensitive linker; a drug-linker conjugate(C) consisting of the combination of a camptothecin-based drug and an enzyme-sensitive linker; and a drug-linker conjugate(D) consisting of the combination of an anti-apoptotic protein inhibitor drug and an enzyme-sensitive linker, in order to select one or more drug-linker conjugates that are homogeneously and symmetrically conjugated to cysteine and lysine residues of the antibody by employing mutually different (i) amino acid residues at the binding site on the antibody, (ii) conjugation sequences, and/or (iii) conjugation methods depending on the drug-linker conjugate. However, the scope of the present invention also encompasses the use of different enzymatically cleavable linkers or the conjugation of two drugs belonging to different classes.

As long as the two drugs have different cytotoxicities, they are classified, respectively, into the camptothecin-based drug and the non-camptothecin-based super-toxic drug categories of the present invention.

For example, the antibody-drug conjugate of the present invention may comprise two types of compounds conjugated thereto, each corresponding to the drug-linker conjugates (B) and (C), respectively.

In this case, the antibody-drug conjugate may be prepared by mixing the selected drug-linker conjugates to form a mixture, and reacting the mixture to a reduced antibody to perform conjugation.

In another embodiment, the antibody-drug conjugate may be prepared by preparing a first reaction mixture by adding the drug-linker conjugate (B) to a reduced antibody at 2- to 4-fold molar equivalents relative to the antibody, and then preparing a second reaction mixture by adding the drug-linker conjugate (C) to the first reaction mixture.

As long as the two drugs have different cytotoxicities, they are classified, respectively, into the camptothecin-based drug and the non-camptothecin-based super-toxic drug categories of the present invention.

The dual-payload ADC (i.e., an antibody-drug conjugate in which two drug-linker conjugates are conjugated to a single antibody) according to the present invention is characterized in that it has a homogeneous structure comprising two types of payloads in a defined ratio, and in that it allows for the flexible introduction of two or more types of payloads to achieve diverse pharmacological effects.

In one embodiment of the present invention, the antibody-drug conjugate (ADC) comprises a first drug-linker conjugate and a second drug-linker conjugate conjugated to a single antibody. The first drug-linker conjugate and the second drug-linker conjugate are each selected from any one of the four groups of drug-linker conjugates (A, B, C, and D). In the present invention, the linkers included in the first and second drug-linker conjugates may be the same or different. For example, the linker included in the first or second drug-linker conjugate may comprise at least one structure selected from the group consisting of GGFG, val-cit and derivatives thereof.

In one embodiment of the present invention, the antibody-drug conjugate (ADC) comprises a first drug-linker conjugate and a second drug-linker conjugate conjugated to a single antibody, wherein the first drug-linker conjugate is the drug-linker conjugate(B) consisting of the combination of a non-camptothecin-based super-toxic drug and an enzyme-sensitive linker, and the second drug-linker conjugate is the drug-linker conjugate(C) consisting of combination of a camptothecin-based drug and an enzyme-sensitive linker. In the present invention, the DAR of the antibody-drug conjugate may be from 4 to 8. In the case where the total DAR is 8, the drugs included in the first and second drug-linker conjugates may be conjugated to the antibody in a ratio of 1:1 to 1:7 (for example, 1:7, 2:6, 3:4, or 4:4); when the total DAR is 6, the ratio may be 1:1 to 1:5 (for example, 1:5, 2:4, 3:3); and when the total DAR is 4, the ratio may be 1:1 to 1:3 (for example, 1:3, 2:2).

The linkers included in the first drug-linker conjugate and the second drug-linker conjugate may be the same or different from each other.

In a preferred embodiment of the present invention, the drug included in the first drug-linker conjugate is an Auristatin-based compound, and the drug included in the second drug-linker conjugate is a Topoisomerase I inhibitor. Herein, the Topoisomerase I inhibitor may be at least one compound or a derivative thereof selected from the group consisting of SN-38, exatecan, Dxd, FL118, and 7-aminoalkyl-substituted camptothecin compounds, but is not limited thereto, and any compound that can strongly inhibit topoisomerase I enzyme in cancer cells and induce cell death may be used without limitation. The drug-to-antibody ratio of the first drug-linker conjugate may be variously adjusted as needed.

The drug-linker conjugate comprising the Topoisomerase I inhibitor as a drug may be represented by any one of Chemical Formula 2 to 5 below.

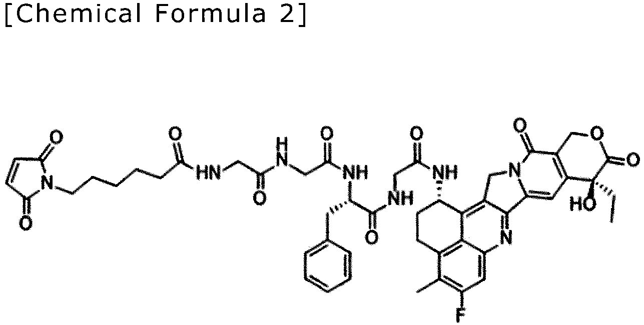

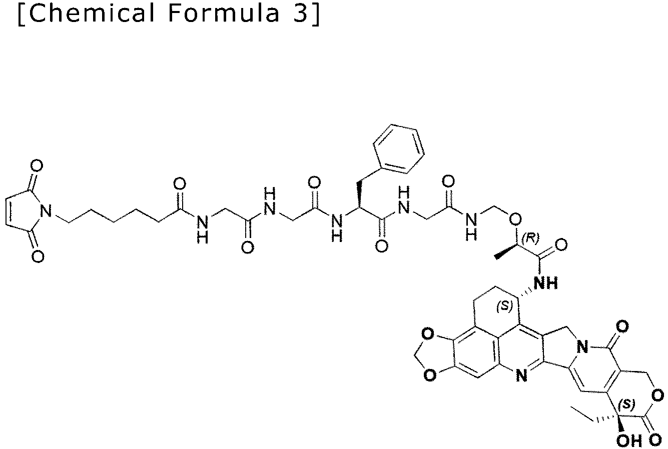

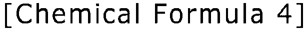

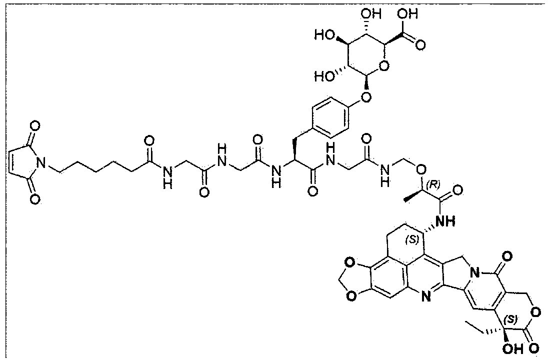

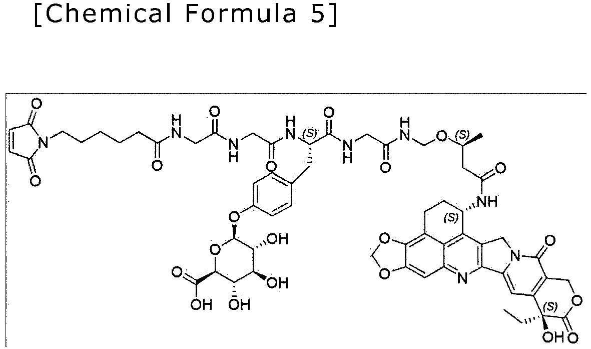

The drug-linker conjugate comprising the Auristatin-based compound as drug may be represented by Chemical Formula 6 or 7 below.

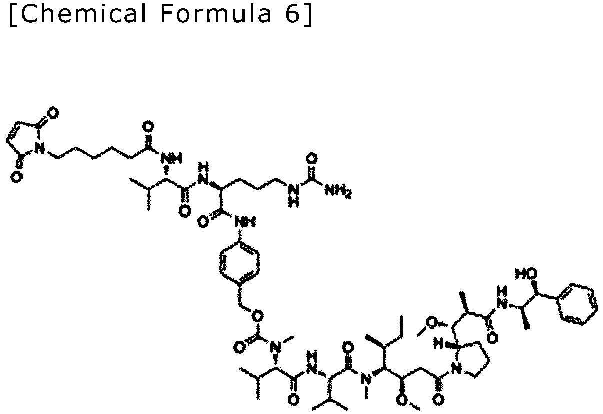

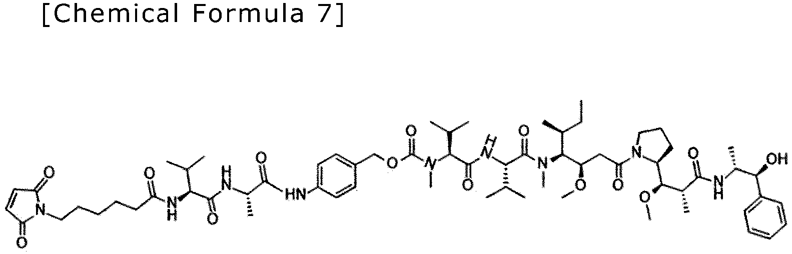

In the present invention, linkers that are readily cleaved by enzymes selectively activated inside cancer cells or in the tumor microenvironment can be used to release the drug, and specific examples include GGFG linkers, AAA linkers, Val-Cit linkers, Val-Ala linkers, and other enzyme-cleavable linkers such as glucuronidase-cleavable linkers and legumain-cleavable linkers. In addition, tandem linkers, which release the drug only after being cleaved by two enzymes, rather than a single enzyme, can also be used.

### [Cytotoxic drugs]

After binding to the target cell, the ADC is internalized into the cell through a process known as receptor-mediated endocytosis. At this stage, it is necessary for a sufficient concentration of active drug to enter the cell. However, the internalization process mediated by the antigen-antibody complex is generally inefficient, and the number of antigens on the cell surface is usually limited to fewer than 1 × 10⁵ receptors/cell. Therefore, the drug used in ADCs must be capable of killing tumor cells even at low concentrations, requiring extremely potent cytotoxic agents. Accordingly, the drugs conjugated to antibodies for use in ADCs are typically 100 to 1,000 times more cytotoxic than conventional chemotherapeutic agents.

Most of the potent cytotoxic drugs used in ADCs exert toxicity not only on cancer cells but also on normal cells due to the bystander effect. Moreover, since the drug must be conjugated in a way that minimally affects the antibody, the amount of payload that can be delivered is limited. This indicates that the cytotoxic drug used in ADCs must be capable of killing most tumor cells at low concentrations (nM or pM range), and that drug release must be controllable while still providing therapeutic effects. Therefore, a wide variety of cytotoxic drugs can be applied as payloads. Such drugs must have low immunogenicity and remain stable during systemic circulation. If the cytotoxic payload is unstable, degradation or modification of the payload may occur during storage or in the conjugated state. Currently approved or clinically evaluated ADCs mainly employ two categories of cytotoxic drugs: microtubule-disrupting agents and DNA-damaging agents. Recently, with the approval of topoisomerase inhibitors for use in ADCs, interest has increased in drugs with diverse mechanisms of action.

The major cytotoxic drugs used in ADCs fall into two categories: microtubule-disrupting agents and DNA-damaging agents. In addition, anti-apoptotic protein inhibitors are also being used.

Microtubules play a critical role in the cell cycle, and disruption of microtubules prevents cell division. Most known microtubule-disrupting agents are derived from natural products and exhibit high cytotoxicity. Unlike microtubule-targeting agents that act at specific phases of the cell cycle, DNA-damaging agents can kill cells at any stage.

Monomethyl auristatin E (MMAE, also referred to as vedotin) is a cytotoxic drug. MMAE binds to microtubules, halts the cell cycle, and induces apoptosis.

Table 1 classifies major cytotoxic drugs used in clinical-stage ADCs according to their mechanisms of action.

**[Table 1]**

| Cytotoxic drugs | Mechanisms of action |
|---|---|
| Auristatins | Tubulin polymerase inhibitor |
| Maytansines | Tubulin depolymerisation |
| Calicheamicins | DNA cleavage |
| Duocarmycins | DNA minor groove alkylating agent |
| PBD dimers | DNA minor groove cross-linker |
| SN-38 | DNA topoisomerase inhibitor |

Representative microtubule-disrupting agents include the auristatin family developed by Seattle Genetics. The cytotoxic drug known as monomethyl auristatin E (MMAE), or vedotin, is a derivative of dolastatin 10, a natural cytotoxic peptide analog isolated from the Indian Ocean Dolabella auricularia, and is a potent inhibitor of microtubule polymerization. This drug is designed to be cleaved and released from the antibody by the enzyme cathepsin B, whereas MMAF, a secondary derivative, is being developed in a form conjugated via a non-cleavable linker, thereby limiting its extracellular release.

Another microtubule inhibitor developed by ImmunoGen is the ansamycin antibiotic derived from the natural product maytansine isolated from the Ethiopian shrub Maytenus serrata. Maytansine binds to tubulin to inhibit microtubule assembly and induces microtubule disassembly, thereby blocking cell division. Maytansine exhibits cytotoxicity in a wide range of tumor cell lines and can suppress tumor growth. These derivatives are designed to be conjugated to linkers via thiol groups. DM1 is readily cleaved under the reducing conditions inside cells, whereas DM4 has a methyl group substituted on its disulfide bond, making it more resistant to reduction.

Since rapidly dividing cells, such as cancer cells, are much more sensitive to cytotoxic agents than slowly dividing normal cells, DNA-damaging agents can be used to selectively eliminate cancer cells. DNA damage is one of the most common mechanisms of action for chemotherapeutic drugs. However, drugs used in clinical settings often exhibit a narrow therapeutic window as their potency increases, resulting in dose-limiting toxicity. Conjugating such highly potent drugs to antibodies with high targeting specificity can enhance both the safety and efficacy of the drug.

Most DNA-damaging agents are also derived from natural products. Bristol-Myers Squibb and Medarex have developed various natural product-based drug substances with DNA-alkylating mechanisms intended to induce mutations. A synthetic toxin known as a duocarmycin analog, developed by Synthon (formerly Syntarga) in the Netherlands, binds to DNA via a similar mechanism. SYD985 (trastuzumab duocarmazine), which has entered phase 3 clinical trials and was developed by Synthon, is known to use duocarmycin.

Other DNA-damaging agents include calicheamicin, pyrrolobenzodiazepine (PBD), and SN-38 (an active metabolite of irinotecan). Calicheamicin, isolated from bacteria by Pfizer, binds to the minor groove of DNA and induces double-strand breaks. Calicheamicin has been used in two of Pfizer's ADCs: Mylotarg and Besponsa. The PBD dimer, a cytotoxic agent developed by Seattle Genetics after their auristatin-based ADC platform, was applied to a CD70-targeting ADC that entered clinical development but was later discontinued. SN-38, the active metabolite of irinotecan, inhibits DNA topoisomerase. Compared to auristatin, maytansinoids, and calicheamicin-cytotoxic drugs used in currently approved ADCs-SN-38 is known to have lower potency, which allows for the development of ADCs with a higher drug-to-antibody ratio while reducing side effects. Representative ADCs using SN-38 as the cytotoxic drug include IMMU-130 and IMMU-132, developed by Immunomedics and currently in clinical trials.

### [camptothecin-based drugs]

Cancer is a disease characterized by indiscriminate cell proliferation and unlimited division. In normal cells, there are mechanisms called cell cycle checkpoints, which inspect for genetic damage and cellular damage during cell division. When DNA damage is detected, repair mechanisms are initiated, or if the damage is severe, apoptosis occurs. However, if such DNA damage is not properly repaired and the cell continues to divide, cancer may arise. A representative example is the BRCA gene, which plays an important role in repairing DNA double-strand breaks (DSBs) through homologous recombination. When DNA damage occurs in the presence of a mutation in the BRCA gene, proper DNA repair does not take place, which increases the frequency of mutations in other genes. In fact, mutations in the BRCA gene significantly increase the incidence of female cancers such as breast and ovarian cancer. Women with BRCA gene mutations are known to have a 5-6-fold higher risk of developing breast cancer and about a 10-fold higher risk of developing ovarian cancer compared to the general population. Through this, it can be understood that the failure to properly repair DNA damage induces genomic instability, thereby leading to cancer.

A common feature of cancer is genomic instability. Although the specific defects in the DNA damage response that give rise to and promote cancer cells are not fully understood, the relationship between DNA damage response deficiency and cancer is indisputable.

For example, approximately 15% of sporadic colorectal tumors exhibit abnormally short or long dinucleotide repeat sequences. These DNA mutations are called microsatellite instability, and are presumed to result from accumulated DNA replication errors due to defective mismatch repair. Microsatellite instability is observed not only in sporadic colorectal tumors but also in hereditary non-polyposis colorectal cancer. Hereditary non-polyposis colorectal cancer is associated with loss-of-function mutations in mismatch repair genes such as MSH2 and MLH1.

Similarly, there are reports that defects in homologous recombination repair can lead to both sporadic and hereditary tumors. According to studies, when 500 samples of high-grade serous ovarian adenocarcinoma were analyzed, 50% showed defects in homologous recombination repair.

The main causes of such defects include mutations and epigenetic silencing in BRCA genes. Other reports indicate that loss-of-function mutations in homologous recombination repair-related genes such as BRCA1, BRCA2, ATM, RAD51C, and RAD51D are found in hereditary breast cancer, endometrial cancer, and pancreatic cancer.

Cancer cells contain defects in the DNA damage response and cannot properly respond to DNA damage. Therefore, substances that induce DNA damage are used as anticancer agents to trigger cell death in cancer cells.

A recent anticancer strategy is to target specific proteins involved in the DNA damage response, and such anticancer agents are referred to as targeted therapies. One example of a targeted therapy is the Topoisomerase inhibitor. The function of topoisomerase is to relieve the supercoiling of DNA by cleaving and re-ligating the phosphodiester backbone of DNA, thereby enabling proper transcription and replication. Topoisomerase inhibitors inhibit this function and leave DNA broken, thereby serving as effective anticancer agents for some cancers such as colorectal cancer and lung cancer.

Topoisomerase I inhibitors (such as irinotecan and topotecan) are anticancer drugs whose efficacy and safety have been verified in clinical settings. They have demonstrated strong anticancer efficacy in various refractory solid tumors, including colorectal, lung, breast, and ovarian cancers. Camptothecin-based drugs such as exatecan and SN-38 have been developed as payloads for ADCs.

Camptothecin is a selective inhibitor of topoisomerase I, an isomerase involved in DNA replication and recombination. It is a natural antitumor alkaloid isolated in 1966 by Wall and colleagues in the United States from the Chinese tree Camptotheca acuminata. Although camptothecin exhibited strong cytotoxicity in vitro, its clinical was initiated by institutions such as U.S. National Cancer Institute (NCI), but was eventually halted due to its extremely poor solubility and associated side effects, such as myelosuppression and hemorrhagic cystitis. However, since the 1990s, interest has reemerged due to its unique mechanism of action, which differs from that of topoisomerase II inhibitors, and its confirmed antitumor efficacy via selective inhibition of topoisomerase I.

DNA topoisomerases are members of the gyrase enzyme family. These are nuclear enzymes that temporarily cleave or unwind the DNA double helix to allow access to genetic material during replication or transcription. They also participate in various intracellular activities such as chromosome condensation, recombination, and DNA repair. The genetic codes for topoisomerase enzymes are highly conserved among different species.

Topoisomerase I, the drug target of camptothecin, has been observed to be upregulated in various malignant tumors. Although this drug does not inhibit the free enzyme, it stabilizes the covalent topo-DNA complex, thereby preventing the re-ligation of cleaved DNA fragments. Accordingly, cellular sensitivity to such topoisomerase-targeting drugs is correlated with the intracellular expression level of the enzyme. By interfering with DNA re-ligation, these drugs prevent the continuation of transcription. A higher expression of Topoisomerase I leads to the formation of more cleavable complexes, resulting in increased drug sensitivity. This has important clinical implications: Topoisomerase I inhibitors can be used to increase the expression of Topoisomerase II, rendering the cells more sensitive to Topoisomerase II inhibitors. This phenomenon is supported by the antagonistic relationship between Topoisomerase I and II. Unlike Topoisomerase II, Topoisomerase I is not closely associated with proliferation in normal tissues. However, in rapidly dividing solid tumors during the S phase of the cell cycle, including colorectal cancer, ovarian cancer, and esophageal cancer-as well as lymphomas-Topoisomerase I is abundantly expressed compared to surrounding normal tissues. It is known that by blocking gene replication and transcription during the S phase, cell death can be induced in tumor cells.

Camptothecin (CPT) exhibits poor aqueous solubility. To prepare for clinical trials, the National Cancer Institute (NCI) synthesized a water-soluble sodium salt form (NSC100880). However, Phase I and II clinical trials could not be completed due to the high toxicity of the compound, including hemorrhagic cystitis, gastrointestinal toxicity (e.g., nausea, vomiting, diarrhea), and myelosuppression, especially leukopenia and thrombocytopenia.

Subsequently, numerous CPT analogs were synthesized to achieve lower toxicity and improved solubility. Among these, two drugs were developed: irinotecan (CPT-11) and topotecan.

Irinotecan (CPT-11), co-developed by Daiichi and Yakult in Japan, became the world's first clinically approved camptothecin-based anticancer drug in 1994. It demonstrated efficacy against lung cancer (both small-cell and non-small-cell types) and was launched in Europe and Japan. In 1995, its efficacy was further verified for colorectal and breast cancers. Topotecan, developed by GlaxoSmithKline, was approved in April 1995 by the U.S. FDA for the treatment of metastatic ovarian cancer and launched thereafter. More recently, a new camptothecin derivative developed domestically, CKD-602 (belotecan), has been shown to be a potent Topoisomerase I inhibitor with water solubility, effectively overcoming the toxicity associated with the poor solubility of earlier camptothecin compounds.

All camptothecin derivatives identified to date contain a core structure composed of five rings, which are essential for cytotoxicity. Structurally, the E-ring, along with the A- and B-rings, has been identified as critical regions. Camptothecin has a pentacyclic structure with a lactone moiety in the E-ring, which is essential for cytotoxicity. In particular, the lactone group at the C-20 position of the E-ring and the α-hydroxy group are important for stabilizing the Topoisomerase I-DNA complex. It has also been demonstrated that modifications to the A- and B-rings can improve water solubility and bioactivity. Modifications to the first ring, for example in the aforementioned drugs, have been shown to enhance solubility and allow for greater drug tolerance.

CKD-602 also introduced a substitution at the B-ring (C-7 position) to improve water solubility and antitumor efficacy. According to Lee et al., CKD-602 exhibited superior antitumor efficacy compared to camptothecin and topotecan across a broad spectrum of cancer cell lines. In addition, in the L1210 leukemia nude mouse model, the maximum tolerated dose (MTD) of CKD-602 was determined to be 25 mg/kg, indicating a favorable safety profile. Commonly known side effects of camptothecin-based drugs are classified into hematologic and non-hematologic toxicities. Hematologic adverse effects include neutropenia with fever, sepsis, and bleeding, while non-hematologic effects include nausea, vomiting, dermatologic adverse effects such as alopecia, and toxicities affecting the gastrointestinal tract, kidneys, and nervous system. According to Kim et al., in animal studies of CKD-602, no significant adverse drug reactions were observed-even at doses more than 10 times higher than the clinically applicable dose-except for increased gastric acid secretion. Furthermore, recent domestic clinical studies have confirmed the relative safety of CKD-602, reporting only reversible and manageable levels of neutropenia and leukopenia, rather than severe systemic toxicity. However, to date, its use remains limited primarily to the treatment in patients who have either failed standard chemotherapy or are unable to undergo standard chemotherapy, that is, to the treatment of refractory or recurrent ovarian and colorectal cancer in patients in whom the disease has relapsed or progressed after standard treatment and who are considered unlikely to benefit further from additional chemotherapy or surgery; to the treatment of refractory or recurrent limited-disease small cell lung cancer in patients who have failed first-line chemotherapy; and to the treatment of extensive-disease small cell lung cancer.

Exatecan, a camptothecin derivative, is a low molecular weight antitumor compound that inhibits Topoisomerase I. Exatecan has been shown to exhibit 5- to 10-fold stronger cellular cytotoxicity compared to SN-38.

Dxd, an Exatecan derivative for ADC use, is a potent Topoisomerase I inhibitor with an IC₅₀ of 0.31 µM, and is employed as the payload of the HER2-targeted ADC (DS-8201a).

FL118 is a potent Topoisomerase I inhibitor that shares the same camptothecin core structure as existing commercialized Top1 inhibitors such as SN-38, topotecan, and exatecan. However, FL118 has demonstrated differentiated antitumor efficacy and superior safety compared to SN-38 in various cancer cell lines and animal models, even as a monotherapy, thereby securing a broader therapeutic window.

Specifically, FL118 shows Top1 inhibitory potency equivalent to or greater than that of SN-38 in cancer cells and exhibits 5- to 20-fold stronger cytotoxicity than SN-38, as indicated by lower IC50 values in various cancer cell lines. Evaluation across 140 cell lines derived from various tumor types also confirmed that FL118 has potent antitumor efficacy, showing IC50 values below 100 nM in the majority of tested cancer cells.

### [Non-camptothecin supertoxin drugs]

In ADCs utilizing existing super toxin such as MMAE, hemiasterlin, calicheamicin, and PBD, stable linker systems have been employed to minimize premature drug release in the bloodstream before reaching tumor tissue. This is a characteristic feature of second-generation ADCs.

Most second-generation ADCs operate by the following mechanism: the antibody portion of the ADC binds to an antigen that is overexpressed on the surface of cancer cells. Upon antigen-antibody interaction, the ADC bound to the antigen is internalized into the cancer cell via endosomes and lysosomes. In the lysosome, the antigen-antibody complex is degraded, and the enzyme (cathepsin B) drug is released, ultimately inducing cancer cell death.

Microtubule-disrupting agents include peptide-like microtubule polymerization inhibitors such as monomethyl auristatin E (MMAE) and MMAF.

PBDs (pyrrolobenzodiazepines), such as SG3199 and SG2057, are DNA-alkylating agents.

Cytotoxic drugs of the PBD class exhibit significantly greater efficacy than conventional cytotoxic agents, but also present toxicity issues upon systemic exposure.

### [Anti-apoptotic protein inhibitor drugs]

Anti-apoptotic proteins such as survivin, cIAP2, and XIAP are major contributors to resistance to anticancer drugs.

Examples of anti-apoptotic protein inhibitors include Bcl-xL inhibitors, survivin inhibitors, MCL-1 inhibitors, and CHK inhibitors.

In general, the mechanism of apoptosis is a series of signaling events in which intracellular proteins are cleaved by proteolytic enzymes called caspases, and multiple types of caspases are known to be involved in the apoptotic process. Caspase-8 is activated by apoptosis-inducing ligands such as TNF-α and Fas ligand and subsequently activates other caspases to induce apoptosis. Meanwhile, during the process of apoptosis, cytochrome c is released through channels in the mitochondrial membrane, and this release is regulated by BCL-2 family proteins that constitute the channels. The released cytochrome c binds with Apaf-1, caspase-9, and dATP, leading to the activation of caspase-9, which in turn activates caspase-3 to induce apoptosis.

Tumor growth is determined by two factors: cell proliferation and cell death. When the tumor cell cycle is arrested by cytotoxic agents, tumor cells undergo apoptosis.

B-cell lymphoma 2(BCL-2) is known to mediate apoptosis.

Although various cancer treatments induce cell death through multiple mechanisms, the activation of apoptosis pathways regulated by BCL-2 is central to the therapeutic effect of both cytotoxic agents and oncogenic kinase inhibitors. However, defects in the mitochondrial apoptosis pathway lead many cancers to develop resistance to cytotoxic drugs.

Overexpression of BCL-2 has been demonstrated in chronic lymphocytic leukemia (CLL) cells, where it mediates tumor cell survival and is associated with resistance to chemotherapy.

Venetoclax is a potent and selective small-molecule inhibitor of the anti-apoptotic protein BCL-2. It is an apoptosis-inducing anticancer agent. Venetoclax binds directly to the BH3-binding groove of BCL-2, displacing pro-apoptotic proteins containing BH3 motifs, such as BIM. As a result, BIM that is not bound to BCL-2 initiates mitochondrial outer membrane permeabilization (MOMP), caspase activation, and apoptosis. In non-clinical studies, venetoclax demonstrated cytotoxicity in tumor cells with BCL-2 overexpression.

Survivin is mainly expressed in cancer cells. Therefore, survivin-targeting compounds can selectively induce apoptosis in cancer cells by binding to and inhibiting survivin, making them promising anticancer agents with a high potential to minimize adverse effects in normal tissues.

### [Linkers]

Among the components that constitute an ADC, the linker is responsible for conjugating the antibody and the cytotoxic drug.

The linker must be stable in the bloodstream to prevent premature separation of the drug from the antibody, maintaining the ADC in a prodrug-like state until it reaches the target site, thereby minimizing damage to normal tissues.

The ideal linker should remain stable during systemic circulation but be cleavable within the target cell to release the cytotoxic drug, allowing the ADC to achieve both efficacy and safety. One of the key challenges in the development of safe and effective ADCs lies in designing an appropriate chemical linker between the cytotoxic drug and the monoclonal antibody. Linker synthesis is often highly complex, and the choice of linker significantly affects the efficiency of drug release.

### 1) Linker Stability

The development of linkers must take into account the long half-life of monoclonal antibodies (mAbs), and the linker must remain stable during systemic circulation. Moreover, the conjugation between the linker and the cytotoxic drug must not adversely affect the stability or pharmacokinetics of the antibody. There have been multiple cases in which ADCs, despite promising preclinical data, failed during clinical development due to the use of inappropriate linkers. In such failed ADCs, it was revealed that the linker released the cytotoxic drug prematurely. As a result, the importance of linker design strategies in ADC development has become increasingly recognized. New ADCs in clinical pipelines that incorporate improved linker technologies have shown the potential for greater safety and efficacy compared to existing ADCs.

In general, catabolic reactions that may occur during systemic circulation involving linker-cytotoxic drug conjugates include the following, although various other unidentified catabolic reactions may also occur.

hydrazone cleavage, protease-mediated dipeptide cleavage, esterase-mediated carbamate cleavage, hydrolysis of acetate esters, disulfide cleavage, and succinimide ring opening.

Catabolic reactions occurring at specific positions of the linker-drug conjugate may, in some cases, retain cytotoxic activity and allow drug activation at the target. However, during systemic circulation, these reactions may also induce off-target toxicity. Conversely, if the cytotoxic activity is lost due to such degradation, even if the drug reaches the target, pharmacological effects may not be achieved. For example, in the case of thailanstatin, developed as a spliceosome inhibitor payload, ester hydrolysis does not abolish activity. In contrast, for tubulin inhibitors such as cryptophycin or tubulysin, ester hydrolysis leads to a loss of activity.

Most ADCs currently in clinical development use a limited variety of chemical linkers, including hydrazone, disulfide, peptide, or thioether linkages.

**[Table 2]**

| linker | Drug release mechanism |
|---|---|
| Hydrazone | Drug release in the acidic environment of the cytosol |
| Peptide | Enzymatic hydrolysis by lysosomal proteases such as cathepsin B |
| Disulfide | Degradation via disulfide exchange with intracellular glutathione thiols |
| Thioether | Degradation via intracellular proteolysis |

As a principle, such chemical linkers are designed to induce the release of cytotoxic drugs inside cancer cells by utilizing differences in intracellular pH, enzyme concentrations, and other factors. Ensuring the in vivo stability of the drug-linker complex after administration remains one of the greatest challenges in ADC development. Chemically unstable linkers such as hydrazone and disulfide are not sufficiently stable in plasma.

In contrast, peptide-based linkers exhibit excellent plasma stability while also providing well-regulated drug-linker stability and controlled drug release. Cleavable dipeptide linkers, such as valine-alanine (Val-Ala) and valine-citrulline (Val-Cit), undergo rapid hydrolysis in the presence of lysosomal extracts or purified human cathepsin B, indicating that their cleavage mechanism is dependent on the intracellular environment.

However, existing linker systems such as Val-Cit or MAC-glucuronide exhibit limited drug delivery efficiency, making it difficult to deliver sufficient amounts of drug.

### 2) Cleavable Linkers and Non-cleavable Linkers

Currently used linkers are generally categorized into cleavable and non-cleavable linkers. Representative cleavable linkers include those that are acidlabile, sensitive to oxidation-reduction reactions, or enzyme-labile, whereas non-cleavable linkers typically include thioether-based linkers (see Table 3).

**[Table 3]**

| Linker type | Mechanism of Action | Example |
|---|---|---|
| Acid-labile | Acidic environment in the cytoplasm | Hydrazone, Silyl ether |
| Cleavable Oxidation-reduction reaction | Redox reaction by intracellular glutathione | Disulfide |
| Enzyme labile | Hydrolyzed by intracellular proteolytic enzymes | Peptide, β-glucuronide |
| Non-cleavable | Degradation of antibodies | Thioehter |

Acid-labile linkers are chemically unstable linkers that were developed during the early stages of ADC research. Although they exhibit low stability, they are still in use today. A representative example is the hydrazone linker, which remains stable at physiological pH (7.3-7.5) in blood, but undergoes hydrolysis and releases the drug in slightly acidic environments such as those surrounding tumor cells (pH 6.5-7.2), endosomes (pH 5.0-6.5), and lysosomes (pH 4.5-5.0) following cellular internalization. However, because acidic conditions are not limited to the tumor microenvironment and can sometimes be found extracellularly, non-specific drug release may occur. Mylotarg^{®}, the first ADC approved by the FDA, utilized a hydrazone linker. Due to its low stability in plasma, it was withdrawn from the U.S. market in 2010 but was reapproved in 2017. Recently, silyl ether linkers have been under investigation. These linkers exhibit high plasma stability and demonstrate a plasma half-life of more than 7 days, which is a significant improvement over hydrazone linkers with a half-life of 2-3 days.

With respect to oxidation-reduction reaction linkers, disulfide linkers are another class of chemically unstable linkers, which function based on redox reactions. Once internalized, the ADC is degraded by enzymatic cleavage of the antibody, followed by linker cleavage through disulfide exchange or reduction by reductants such as glutathione, leading to drug release. Glutathione is a low-molecular-weight thiol known as an antioxidant that regulates cell proliferation and death, and protects cells from oxidative stress and inflammation. While glutathione is present in the intracellular space at concentrations of 0.5-10 mM, it can reach levels up to 1,000 times higher in hypoxic tumor environments. In contrast, it exists in plasma at relatively low concentrations (2-20 µM), thus providing disulfide linkers with high plasma stability. This minimizes non-specific drug release and enables tumor-selective drug activation with relative safety.

Regarding enzyme-labile linkers, peptide linkers are composed of dipeptides or tetrapeptides that are recognized and cleaved by lysosomal proteases once the ADC is internalized. Tetrapeptide linkers were used during early development but showed limitations such as relatively slow drug release and the tendency to aggregate when conjugated with hydrophobic drugs. These issues were resolved through the development of dipeptide linkers such as Val-Cit, Phe-Lys, Val-Lys, and Val-Ala, which have been successfully applied to ADCs such as Adcetris^{®} and Vedotin^{®}.

The β-glucuronide linker was introduced in 2006 by Seattle Genetics and is cleaved by β-glucuronidase, a lysosomal carbohydrate-degrading enzyme, to release cytotoxic drugs. β-glucuronidase is abundantly present in lysosomes and is known to be overexpressed in certain tumors. This enzyme shows high enzymatic activity under acidic conditions but retains only about 10% of its activity at neutral pH. Due to this property, ADCs incorporating β-glucuronide linkers exhibit improved plasma stability and prevent premature drug release in non-target tissues. To evaluate the plasma stability of the β-glucuronide linker, a comparative study was conducted in mouse plasma alongside the Val-Cit linker. After 7 days, the β-glucuronide linker retained 89% of its integrity, whereas the Val-Cit linker retained less than 50%, indicating that the β-glucuronide linker is significantly more stable. The respective half-lives were measured to be approximately 81 days and 6 days. Moreover, ADCs with β-glucuronide linkers demonstrated high stability and efficacy even when conjugated with high drug loads (up to eight).

A representative non-cleavable linker is the thioether linker, which exhibits higher plasma stability compared to cleavable linkers. Unlike cleavable linkers, non-cleavable linkers do not undergo degradation themselves. Therefore, drug release can only occur after the antibody is degraded inside the cell following internalization of the ADC.

The released drug typically carries a charge, which limits its diffusion to surrounding cells and reduces the potential for bystander effects. Although bystander toxicity is absent, the cytotoxic effect is confined to the target cell following internalization, suggesting relatively improved safety. This also indicates that ADCs employing non-cleavable linkers rely more heavily on intracellular biological mechanisms within the target cells. Numerous studies have shown that ADCs with non-cleavable linkers exhibit high stability and efficacy, making them attractive linker candidates in ADC development. An example of such a technology is Kadcyla^{®}.

In the case of brentuximab vedotin (Adcetris), it incorporates a cleavable Val-Cit dipeptide linker, which is selectively cleaved by cathepsin B present in the lysosomes of target cancer cells, thereby releasing MMAE. It remains stable during systemic circulation. ADCs containing cleavable dipeptide linkers such as Val-Ala or Val-Cit bind to antigens of target cancer cells via the antibody region of the ADC, forming ADC-antigen complexes. These complexes are then internalized through the endosomal-lysosomal pathway. In such cases, intracellular drug release is governed by the internal environment of the endosomes or lysosomes. For instance, hydrazone linkers are hydrolyzed in acidic conditions to release the drug, while Val-Cit dipeptide linkers release MMAE upon cleavage by lysosomal proteases such as cathepsin B.

In contrast, ado-trastuzumab emtansine (Kadcyla) contains a non-cleavable SMCC linker. With non-cleavable linkers, the cytotoxic drug is only released following lysosomal degradation of the internalized ADC, thereby minimizing unwanted drug release in the systemic circulation. Furthermore, the chemical nature of the conjugated drug can be modified through non-cleavable linkers to adjust its carrier affinity or enhance its efficacy.

The stability of an ADC during transport to the target site is critically important for achieving the desired therapeutic outcomes, regardless of whether a cleavable or non-cleavable linker is used.

### [click Reaction]

In the present invention, the linkage between the [linker]-[antibody] may be formed via a "click" reaction of Reaction Formula 1, wherein a thiol group present in the antibody reacts with the maleimide group or maleic hydrazide group of an acid-sensitive linker.

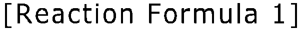

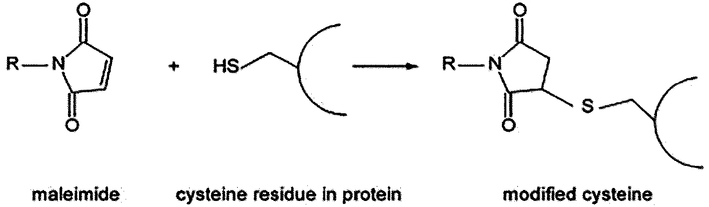

### [Hydrophilic Spacer]

Hydrophilic linkers containing sulfonate or PEG, which exhibit high solubility in both organic solvents and aqueous media, address multiple issues observed with hydrophobic linkers. PEG linkers offer advantages such as water solubility, low toxicity, low immunogenicity, and tunable linker chain lengths. In this regard, the use of PEG linkers has been reported to significantly improve the in vivo pharmacokinetic profile, with increased half-life and plasma concentration, thereby increasing the area under the plasma concentration-time curve (AUC).

### [Enzyme-Sensitive Linker]

In one embodiment of the present invention, the enzyme-sensitive linker may be -S-Maleimide-Spacer-Enzymatic cleavable site-Self-immolative spacer-(Payload) or -S-Dibromomaleimide-Spacer-Enzymatic cleavable site-Self immolative spacer-(Payload).

In vivo transformation by linker deconjugation may occur via chemical degradation or enzymatic cleavage.

In the case of an ADC in which the linker is conjugated via an amide bond to a lysine residue, enzymatic amide hydrolysis may lead to the release of the linker-cytotoxic drug moiety. Additionally, in ADCs where the linker contains a maleimide group or a disulfide bond and is conjugated to a cysteine residue, the sulfur atom of the cysteine may undergo reduction, resulting in an exchange reaction by which the conjugated linker-cytotoxic drug is cleaved off. Following deconjugation, the cysteine residue of the monoclonal antibody may exist in a state where it forms disulfide bonds with other cysteine amino acids or endogenous/exogenous thiol-containing substances such as glutathione (GSH). Accordingly, the ADC may lose its mechanism of action at the target due to the loss of the cytotoxic drug, while the released linker-cytotoxic drug moiety may form adducts with other proteins or enzymes, or undergo metabolic transformation to exert activity, thereby potentially causing toxicity.

### [Acid-Sensitive Linker]

In the present invention, the term "acid-sensitive linker" refers to a linker that is stable under the neutral pH of blood (pH 7.3 ~ 7.5), but undergoes hydrolysis and releases the drug under mildly acidic environments such as in the tumor microenvironment (pH 6.5 ~ 7.2), endosomes (pH 5.0 ~ 6.5), and lysosomes (pH 4.5 ~ 5.0) following intracellular internalization. Therefore, the acid-sensitive linker in the present invention comprises a hydrophilic molecular structure to create a hydrolytic environment. To this end, the acid-sensitive linker may include a polyethylene glycol (PEG) spacer.

In order for an acid-sensitive linker to degrade under acidic conditions (pH ≤ 7) and release a free camptothecin-based drug, the camptothecin drug and the acid-sensitive linker are preferably connected via a carbonate or ester bond.

Tetrapeptide linkers have shown limitations due to potential ADC aggregation when combined with hydrophobic drugs.

The linker CL2A, used in the FDA-approved ADC Trodelvy, satisfies (i) stability during storage after synthesis, (ii) in vivo stability with minimal free payload exposure in plasma during administration, and (iii) rapid release of the payload in tumor tissues.

In the first-generation CL2 derivative, a Phe-Lys peptide was introduced to allow cathepsin B cleavage. To simplify the synthesis process, phenylalanine was removed from CL2A, thereby eliminating the cathepsin B cleavage site. This modification did not affect conjugation binding efficiency, stability, or efficacy. This suggests that the primary mechanism of payload release in CL2 is not cleavage by cathepsin B, but rather pH-sensitive cleavage of a benzyl carbonate bond on the lactone ring of SN-38.

In the present invention, the acid-sensitive linker used may include the CL2A linker and be designed as shown in Chemical Formula 1 below to enable selective and efficient delivery of a camptothecin-based drug to tumor tissues. That is, the acid-sensitive linker in the present invention may be derived from the compound represented by Chemical Formula 1 below. 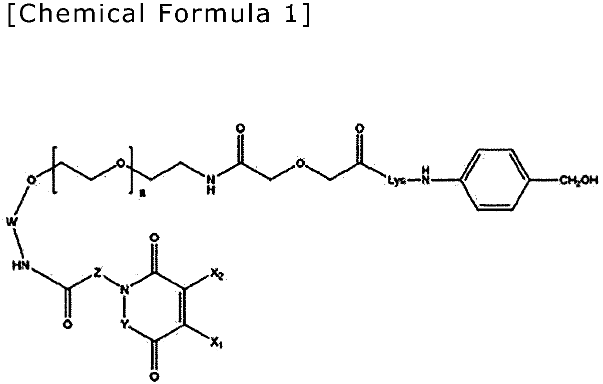
Herein, X₁ and X₂ are each independently -H or halogen;
Y is -NH-, -NR_{A}-, or a direct bond;
Z is -C1-C4 alkylene, -C3-C6 cycloalkylene, -(C1-C2 alkylene)-(C3-C6 cycloalkylene)-, -(C3-C6 cycloalkylene)-(C1-C2 alkylene)-, or -(C1-C2 alkylene)-(C3-C6 cycloalkylene)-(C1-C2 alkylene);
W is -R_{B}-, -M-R_{B}-M-, -M-R_{B}-, or -R_{B}-M-R_{C}-;
R_{A} to R_{C} are each independently C1-C4 alkylene;
M is ; and 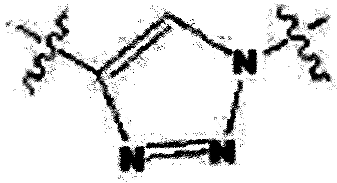
n is an integer from 5 to 9.
Preferably, in the above Chemical Formula 1,
X₁ and X₂ are each independently -H or halogen;
Y is -NR_{A}- or a direct bond;
Z is -C1-C4- alkylene, -(C1-C2 alkylene)-(C3-C6 cycloalkylene)-, or -(C3-C6 cycloalkylene)-(C1-C2 alkylene);
W is -R_{B}- or -R_{B}-M-R_{C}-;
R_{A} to R_{C} are each independently C1-C4 alkyl;
M is ; and 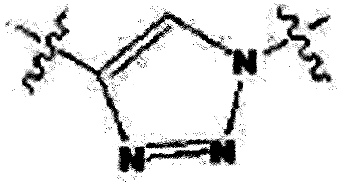
n is an integer from 5 to 9.

In the present invention, the length of the linker, i.e., the value of n in Chemical Formula 1, may be an integer from 5 to 9, specifically from 6 to 8, and more specifically 7; however, it is not limited thereto. Even in cases where n falls outside the above range, if the change in linker length does not result in any significant difference in effect, such variations are naturally considered to fall within the scope of equivalents of the present invention.

Since the aqueous dispersibility of the drug can be improved by positioning a polyethylene glycol (PEG) spacer between the drug and the carrier, the linker of Chemical Formula 1 comprises a low molecular weight PEG moiety including a defined number (n = 5 to 9) of PEG monomers.

The acid-sensitive linker of Chemical Formula 1 is a customized linker that can be optimized depending on the characteristics of the target, the payload, and the carrier.

Camptothecin-based drugs possess various sites that are suitable for attachment with different linkers for the preparation of carrier-drug conjugates. For example, the hydroxyl group of camptothecin-based drugs may be used as the site for attachment to the linker.

Accordingly, in the present invention, the [camptothecin-based drug]-[acid-sensitive linker] may be formed by linking the hydroxyl group of the camptothecin-based drug to the hydroxyl group site of the acid-sensitive linker represented by Chemical Formula 1.

### [Mechanism of Action of an ADC comprising a drug-linker conjugate (A) consisting of the combination of a camptothecin-based drug and an acid-sensitive linker]

As used herein, the term "immunoconjugate" refers to a complex in which a cytotoxic drug-linker conjugate is attached to an antibody or an antigen-binding fragment thereof, and falls within the category of antibody-drug conjugates (ADCs) of the present invention.

Since ADCs are a type of immunoconjugate, descriptions of ADCs and immunoconjugates may be used interchangeably herein.

Upon administration in vivo, the immunoconjugate binds to the antigen targeted by the antibody or antigen-binding fragment thereof, and releases the drug, allowing the drug to act on the target cell and/or surrounding cells, thereby achieving excellent therapeutic efficacy with reduced side effects as a targeted drug.

Factors that critically influence the efficacy of immunoconjugates include particularly (1) drug potency, (2) drug-linker stability, and (3) efficient on-target drug release. Due to the complex interplay of these multiple factors, predicting the overall efficacy of an immunoconjugate, which is a combination of these individual elements, based solely on known information for each factor, is extremely difficult.

If the immunoconjugate is designed to release the drug only after internalization, and the internalization process is inefficient, then a sufficient concentration of active drug may not be delivered into the cell. In addition, if a hydrophobic drug is selected as the cytotoxic drug, bystander cell-killing effects on neighboring cells may be limited.

To overcome these issues, the immunoconjugate of the present invention
(i) uses an acid-sensitive linker to release a hydrophobic drug capable of penetrating the cell membrane and functioning within the cell in the tumor microenvironment surrounding the cancer (pH ≤7), thereby allowing the intracellular delivery of a large amount of free-form hydrophobic drug;
(ii) is characterized by the use of a hydrophobic camptothecin-based drug capable of penetrating the cell membrane, in order to release the drug upon cleavage of the acid-sensitive linker in the acidic environment (pH ≤ 7) surrounding the cancer cells and to allow the accumulation of a large amount of free-form drug within the cell.

Camptothecin-based drugs are hydrophobic small molecules capable of penetrating cell membranes. Therefore, they can rapidly accumulate in tumor tissues, maintain high concentrations for extended periods, diffuse into cells, exert cytotoxic effects and induce cell death, and subsequently be released to continuously act on surrounding cells by re-entering through the cell membranes.

As described above, the immunoconjugate of the present invention is characterized by the combination of a camptothecin-based drug and an acid-sensitive linker. Thus, depending on the intended purpose, an arbitrary antibody or antigen-binding fragment thereof may be conjugated to construct an immunoconjugate, and all such variations are encompassed within the scope of the present invention. For example, for enhanced anticancer efficacy, immunoconjugates can be prepared by conjugating the camptothecin-based drug-acid-sensitive linker conjugate of the present invention with trastuzumab, cetuximab, or sacituzumab.

According to the present invention, the antibody to which the camptothecin-based drug is linked via an acid-sensitive linker follows the same initial mechanism as second-generation ADCs, wherein the antibody binds to an antigen overexpressed on the surface of cancer cells. However, while some portion undergoes intracellular processing like second-generation ADCs, a significant portion can release the drug in response to the acidic tumor microenvironment surrounding the cancer cells. The released drug then enters the cancer cells via passive diffusion, bypassing endosomal or lysosomal pathways and acting without enzymatic cleavage (e.g., cathepsin B), directly inducing apoptosis. Compared to second-generation ADCs that require enzymatic activation, the immunoconjugates of the present invention exhibit the same level of target antigen specificity but offer enhanced drug release and improved intracellular delivery efficiency to cancer cells via pH-sensitive linkers.

Furthermore, although the linker is required to remain stable in the bloodstream to prevent premature dissociation of the drug from the antibody and to maintain a prodrug state until it reaches the target, the present invention employs an acid-sensitive linker having a hydrophilic molecular structure that creates a hydrolytic environment. This design mitigates the problem of ADC aggregation that may occur when conjugated with a hydrophobic drug.

Different linker types yield distinct catabolites. In this regard, the [camptothecin-based drug]-[acid-sensitive linker] of the present invention is preferably connected via a carbonate or ester bond, such that a free form of the camptothecin-based drug is released upon cleavage of the acid-sensitive linker.

Although carbamate bonds are generally known for higher linker stability, the present invention intentionally employs less stable ester or carbonate bonds to enable camptothecin-based drug release both in the extracellular acidic tumor environment and inside cancer cells.

Blood maintains a constant pH of approximately 7.3 to 7.4. Accordingly, the acid-sensitive linker does not undergo cleavage in the bloodstream, and even if cleavage occurs, the release rate of the camptothecin-based drug from the ADC at the neutral pH of serum is significantly lower than that observed in the acidic environment of tumor tissues.

In the present invention, the immunoconjugate may have a drug-to-antibody ratio (DAR) of 4 to 8, and preferably DAR 4 or 8.

### [Mechanism of Action of ADCs Comprising the Drug-Linker Conjugate(A) consisting of the combination of a camptothecin-based Drug and an acid-sensitive linker; and the Drug-Linker Conjugate(B) consisting of the combination of a non-camptothecin-based super-Toxic drug and an Enzyme-sensitive linker]

Rapid drug release from Group A enables a first-line attack against cancer cells, followed by a second-line cytotoxic assault through the delayed but potent release of super toxins from Group B, thereby promoting complete eradication of cancer cells. For example, in the case of super toxins such as MMAE and Hemiasterlin, a drug-to-antibody ratio (DAR) of more than 4 cannot be used in ADC production due to toxicity; however, MMAE can be used at a DAR of 4 or less, and the insufficient cytotoxicity can be compensated by a TOP1 inhibitor, which is a camptothecin-based drug.

All disulfide bonds exposed on the exterior of a single antibody can be conjugated with drug-linkers of Group A and Group B, and in such a case, the DAR (drug-to-antibody ratio) may be 8 (Group A: 4-7, Group B: 1-4), with a non-limiting example being (MMAE-Cit-Val)2-Trastuzumab-(CL2A-FL118)6.

### [Mechanism of Action of an ADC comprising a drug-linker conjugate(A) consisting of the combination of a camptothecin-based drug and an acid-sensitive linker; and a drug-linker conjugate(C) consisting of the combination of a camptothecin-based drug and an enzyme-sensitive linker]

Using two types of linker systems, it is possible to configure different drug release rates for the same payload (or payloads having similar functions).

Rapid drug release proceeds from Group A, which releases the drug efficiently in the tumor microenvironment, while drug release from Group C occurs at a relatively slower rate, thereby allowing time-staggered killing of cancer cells.

All disulfide bonds exposed on the exterior of a single antibody can be conjugated with the drug-linkers of Group A and Group C, and in such a case, the DAR may be 8 (Group A: 1-7, Group C: 1-7), with a non-limiting example being (Dxd-GFGG)4-Trastuzumab-(CL2A-Dxd)4.

### [Mechanism of Action of an ADC comprising a drug-linker conjugate(B) consisting of the combination of a non-camptothecin-based super-toxic drug and an enzyme-sensitive linker; and a drug-linker conjugate(C) consisting of the combination of a camptothecin-based drug and an enzyme-sensitive linker]

Both Group B and Group C can be delivered into cancer cells at relatively slow (sustained) rates. Thus, the disadvantage of super toxins, which cannot be used at a DAR greater than 4 due to toxicity, can be compensated with a TOP1 inhibitor. Group B and Group C can be competitively delivered into cancer cells to induce cell death.

All disulfide bonds exposed on the exterior of a single antibody can be conjugated with drug-linkers of Group B and Group C, and in such a case, the ADC may have a DAR of 8 (Group B: 1-4, Group C: 4-7), with a non-limiting example being (MMAE-Cit-Val)2-Trastuzumab-(GGFG-Dxd)6.

### [Mechanism of Action of an ADC comprising a drug-linker conjugate(B) consisting of the combination of a non-camptothecin-based super-toxic drug and an enzyme-sensitive linker; and a drug-linker conjugate(D) consisting of the combination of an anti-apoptotic protein inhibitor and an enzyme-sensitive linker]

Both Group B and Group D can be delivered into cancer cells at relatively slow (sustained) rates. Thus, the disadvantage of super toxins, which cannot be used at a DAR greater than 4 due to toxicity, can be compensated with an anti-apoptotic protein inhibitor. Group B and Group D can be competitively delivered into cancer cells to induce cell death.

In addition, the use of Group D, which can enhance the effect of the drug in Group B, can induce a potent anticancer effect. Group B and Group D may be adjusted in a ratio of 1:2 or 1:4, and the drug-linkers can be conjugated to all disulfide bonds exposed on the exterior of a single antibody. A non-limiting example is (MMAE-Cit-Val)2-Mirzotamab-(Val-Ala-Clezutoclax)4.

### [Targeted antigen]

To deliver a highly cytotoxic drug specifically to a target cancer cell, identifying the target antigen is the first critical step in developing an ADC. The use of antibodies allows for high specificity to the target and a long half-life enabling prolonged systemic circulation. This in turn enables selective accumulation of the cytotoxic drug in tumor cells while minimizing exposure to normal tissues, thereby reducing off-target damage and side effects and enhancing therapeutic efficacy. For this reason, the target antigen must be selected based on the following criteria. First, the target antigen must be uniformly overexpressed on the surface of tumor cells, while being minimally or not expressed in normal cells. A representative example is the Human Epidermal Growth Factor Receptor 2 (HER2), which is known to be overexpressed more than 100-fold in HER2-positive breast cancer compared to normal cells. Prior to generating antibodies, various profiling techniques are used to analyze tumor-specific expression of candidate target antigens, and when overexpression of a specific antigen is confirmed, a monoclonal antibody recognizing the antigen is generated. Second, the binding affinity to the antigen is important. Due to the nature of antibodies that undergo receptor-mediated internalization, higher binding strength to the epitope of the antigen results in increased internalization, thereby enhancing therapeutic efficacy. Additionally, low immunogenicity is a desirable property. First-generation ADCs were produced using murine antibodies, which when administered to humans, caused immune responses and antibody neutralization against the mouse-derived antibody, thereby hindering anticancer efficacy. These immunogenicity-related issues were largely resolved by the development of chimeric antibodies, humanized antibodies, and fully human antibodies through advances in genetic engineering technology.

For effective delivery, once the antibody recognizes the tumor antigen, internalization of the drug-conjugated antibody into the cancer cell must occur. To enhance cellular internalization in cancer cells, bispecific antibodies are under development.

MEDI4267 (Trastuzumab-META), developed by MedImmune and AstraZeneca, is a biparatopic antibody targeting two non-overlapping epitopes on HER2. This design induces HER2 receptor clustering, thereby promoting internalization, lysosomal trafficking, and degradation.

Moreover, bispecific antibodies incorporating a tumor-targeting antibody along with a lysosomal marker such as CD63, APLP2, and prolactin receptor have been shown to enhance internalization compared to single antibodies, while simultaneously recognizing tumor-associated antigens. There exists a bispecific ADC composed of trastuzumab targeting HER2 and CD63 (HER2xCD63-duostatin-3, developed by Creative Biolabs), which demonstrated potent anticancer efficacy by reducing off-target delivery to normal tissues and enabling tumor cell-specific delivery.

In designing the antibody-drug conjugate (ADC) or immunoconjugate of the present invention, the target may be expanded not only to cancer cells, but also to infectious disease organisms and/or cells associated with autoimmune diseases.

Accordingly, the cell targeted by the antibody or a fragment thereof containing the antigen-binding portion may be a cancer cell, an infectious disease organism, and/or a cell related to an autoimmune disease.

Non-limiting examples of target antigens include those selectively distributed on the surface of cancer cells such as Her2, FolR, and PSMA, as well as cancer-overexpressed antigens such as Trop2, which may be sparsely present in normal tissues.

The cancer cell target antigen may be, for example, 5T4, ABL, ABCF1, ACVR1, ACVR1B, ACVR2, ACVR2B, ACVRL1, ADORA2A, AFP, Aggrecan, AGR2, AICDA, AIF1, AIGI, AKAP1, AKAP2, ALCAM, ALK, AMH, AMHR2, ANGPT1, ANGPT2, ANGPTL3, ANGPTL4, ANPEP, APC, APOCI, AR, aromatase, ASPH, ATX, AX1, AXL, AZGP1 (zinc-α-glycoprotein), B4GALNT1, B7, B7.1, B7.2, B7-H1, B7-H3, B7-H4, B7-H6, BAD, BAFF, BAG1, BAI1, BCR, BCL2, BCL6, BCMA, BDNF, BLNK, BLR1 (MDR15), BIyS, BMP1, BMP2, BMP3B (GDFIO), BMP4, BMP6, BMP8, BMP10, BMPR1A, BMPR1B, BMPR2, BPAG1 (plectin), BRCA1, C19orfIO (IL27w), C3, C4A, C5, C5R1, CA6, CA9, CANT1, CAPRIN-1, CASP1, CASP4, CAV1, CCBP2 (D6/JAB61), CCL1 (1-309), CCLI1 (eotaxin), CCL13 (MCP-4), CCL15 (MIP-Id), CCL16 (HCC-4), CCL17 (TARC), CCL18 (PARC), CCL19 (MIP-3b), CCL2 (MCP-1), MCAF, CCL20 (MIP-3a), CCL21 (MEP-2), SLC, exodus-2, CCL22(MDC/STC-I), CCL23 (MPIF-I), CCL24 (MPIF-2/ eotaxin-2), CCL25 (TECK), CCL26(eotaxin-3), CCL27 (CTACK/ILC), CCL28, CCL3 (MIP-Ia), CCL4 (MIPIb), CCL5(RANTES), CCL7 (MCP-3), CCL8 (mcp-2), CCNA1, CCNA2, CCND1, CCNE1, CCNE2, CCR1 (CKR1/HM145), CCR2 (mcp-IRB/RA), CCR3 (CKR3/CMKBR3), CCR4, CCR5(CMKBR5/ChemR13), CCR6 (CMKBR6/CKR-L3/STRL22/DRY6), CCR7 (CKR7/EBI1), CCR8 or CDw198 (CMKBR8/TERI/CKR-L1), CCR9 (GPR-9-6), CCRL1 (VSHK1), CCRL2 (L-CCR), CD13, CD164, CD19, CDH6, CDIC, CD2, CD20, CD21, CD200, CD22, CD23, CD24, CD27, CD28, CD29, CD3, CD33, CD35, CD37, CD38, CD3E, CD3G, CD3Z, CD4, CD40, CD40L, CD44, CD45RB, CD47, CD52, CD56, CD69, CD70, CD72, CD74, CD79A, CD79B, CD8, CD80, CD81, CD83, CD86, CD97, CD99, CD117, CD125, CD137, CD147, CD179b, CD223, CD279, CD152, CD274, CDH1 (E-cadherin), CDH1O, CDH12, CDH13, CDH18, CDH19, CDH2O, CDH3, CDH5, CDH7, CDH8, CDH9, CDH17, CDK2, CDK3, CDK4, CDK5, CDK6, CDK7, CDK9, CDKN1A (p21Wap1/Cip1), CDKN1B (p27Kip1), CDKN1C, CDKN2A (p16INK4a), CDKN2B, CDKN2C, CDKN3, CEA, CEACAM5, CEACAM6, CEBPB, CERI, CFC1B, CHGA, CHGB, Chitinase, CHST1O, CIK, CKLFSF2, CKLFSF3, CKLFSF4, CKLFSF5, CKLFSF6, CKLFSF7, CKLFSF8, CLDN3, CLDN6, CLDN7 (claudin- 7), CLDN18, CLEC5A, CLEC6A, CLEC11A, CLEC14A, CLN3, CLU (clusterin), CMKLR1, CMKOR1 (RDC1), CNR1, C-MET, COL18A1, COLIA1, COL4A3, COL6A1, CR2, Cripto, CRP, CSF1 (M-CSF), CSF2 (GM-CSF), CSF3 (GCSF), CTAG1B (NY-ESO-1), CTLA4, CTL8, CTNNB1 (b-catenin), CTSB (cathepsin B), CX3CL1 (SCYD1), CX3CR1 (V28), CXCL1 (GRO1), CXCL1O (IP-IO), CXCLI1 (1-TAC/IP-9), CXCL12 (SDF1), CXCL13, CXCL14, CXCL16, CXCL2 (GRO2), CXCL3 (GRO3), CXCL5 (ENA-78/LIX), CXCL6 (GCP-2), CXCL9 (MIG), CXCR3 (GPR9/CKR-L2), CXCR4, CXCR6 (TYMSTR/STRL33/Bonzo), CYB5, CYC1, CYSLTR1, DAB2IP, DES, DKFZp45110118, DLK1, DNCL1, DPP4, E2F1, Engel, Edge, Fennel, EFNA3, EFNB2, EGF, EGFR, ELAC2, ENG, Enola, ENO2, ENO3, EpCAM, EPHA1, EPHA2, EPHA3, EPHA4, EPHA5, EPHA6, EPHA7, EPHA8, EPHA9, EPHA10, EPHB1, EPHB2, EPHB3, EPHB4, EPHB5, EPHB6, EPHRIN-A1, EPHRIN-A2, EPHRINA3, EPHRIN-A4, EPHRIN-A5, EPHRIN-A6, EPHRIN-B1, EPHRIN-B2, EPHRIN-B3, EPHB4, EPG, ERBB2 (HER-2), ERBB3, ERBB4, EREG, ERK8, estrogen receptor, Earl, ESR2, F3 (TF), FADD, FAP, farnesyltransferase, FasL, FASNf, FCER1A, FCER2, FCGR3A, FGF, FGF1 (aFGF), FGF10, FGF1 1, FGF12, FGF12B, FGF13, FGF14, FGF16, FGF17, FGF18, FGF19, FGF2 (bFGF), FGF20, FGF21, FGF22, FGF23, FGF3 (int-2), FGF4 (HST), FGF5, FGF6 (HST-2), FGF7 (KGF), FGF8, FGF9, FGFR1, FGFR2, FGFR3, FGFR4, FIGF (VEGFD), FIL1(EPSILON), FBL1 (ZETA), FLJ12584, FLJ25530, FLRT1 (fibronectin), FLT1, FLT-3, FOLR1, FOS, FOSL1(FRA-1), FR-α, FY (DARC), GABRP (GABAa), GAGEB1, GAGEC1, GALNAC4S-6ST, GATA3, GD2, GD3, GDF5, GFI1, GFRA1, GGT1, GMCSF, GNAS1, GNRH1, GPC1, GPC3, GPNB, GPR2 (CCR10), GPR31, GPR44, GPR81 (FKSG80), GRCC1O (C1O), GRP, GSN (Gelsolin), GSTP1, GUCY2C, HAVCR1, HAVCR2, HDAC, HDAC4, HDAC5, HDAC7A, HDAC9, Hedgehog, HER3, HGF, HIF1A, HIP1, histamine and histamine receptor, HLA-A, HLA-DR, HLA-DRA, HLA-E, HM74, HMOXI, HSP90, HUMCYT2A, ICEBERG, ICOSL, ID2, IFN-a, IFNA1, IFNA2, IFNA4, IFNA5, EFNA6, BFNA7, IFNB1, IFNy, IFNW1, IGBP1, IGF1, IGFIR, IGF2, IGFBP2, IGFBP3, IGFBP6, DL-1, ILIO, ILIORA, ILIORB, IL-1, IL1R1 (CD121a), IL1R2(CD121b), ILIRA, IL-2, IL2RA (CD25), IL2RB(CD122), IL2RG(CD132), IL-4, IL-4R(CD123), IL-5, IL5RA(CD125), IL3RB(CD131), IL-6, IL6RA, (CD126), IR6RB(CD130), IL-7, IL7RA(CD127), IL-8, CXCR1 (IL8RA), CXCR2, (IL8RB/CD128), IL-9, IL9R(CD129), IL-10, IL10RA(CD210), IL10RB(CDW210B), IL-11, IL11RA, IL-12, IL-12A, IL-12B, IL-12RB1, IL-12RB2, IL-13, IL13RA1, IL13RA2, IL14, IL15, IL15RA, IL16, IL17, IL17A, IL17B, IL17C, IL17R, IL18, IL18BP, IL18R1, IL18RAP, IL19, ILIA, ILIB, ILIF10, ILIF5, IL1F6, ILIF7, IL1F8, DL1F9, ILIHYI, ILIR1, IL1R2, ILIRAP, ILIRAPLI, ILIRAPL2, ILIRL1, IL1RL2, ILIRN, IL2, IL20, IL20RA, IL21R, IL22, IL22R, IL22RA2, IL23, DL24, IL25, IL26, IL27, IL28A, IL28B, IL29, IL2RA, IL2RB, IL2RG, IL3, IL30, IL3RA, IL4, 1L4, IL6ST (glycoprotein 130), ILK, INHA, INHBA, INSL3, INSL4, IRAK1, IRAK2, ITGA1, ITGA2, ITGA3, ITGA6 (α6 integrin), ITGAV, ITGB3, ITGB4 (α4 integrin), JAG1, JAK1, JAK3, JTB, JUN, K6HF, KAI1, KDR, KIT, KITLG, KLF5 (GC Box BP), KLF6, KLK10, KLK12, KLK13, KLK14, KLK15, KLK3, KLK4, KLK5, KLK6, KLK9, KRT1, KRT19 (keratin 19), KRT2A, KRTHB6 (hair-specific type II keratin), L1CAM, LAG3, LAMA5, LAMP1, LEP (leptin), Lewis Y antigen ("LeY"), LILRB1, Lingo-p75, Lingo-Troy, LGALS3BP, LRRC15, LPS, LTA (TNFb), LTB, LTB4R (GPR16), LTB4R2, LTBR, LY75, LYPD3, MACMARCKS, MAG or OMgp, MAGEA3, MAGEA6, MAP2K7 (c-Jun), MCP-1, MDK, MIB1, midkine, MIF, MISRII, MJP-2, MLSN, MK, MKI67 (Ki-67), MMP2, MMP9, MS4A1, MSMB, MT3 (metallothionein-UI), mTOR, MTSS1, MUC1 (mucin), MUC16, MYC, MYD88, NCK2, NCR3LG1, neurocan, NFKBI, NFKB2, NGFB (NGF), NGFR, NgR-Lingo, NgRNogo66, (Nogo), NgR-p75, NgR-Troy, NMEI (NM23A), NOTCH, NOTCH1, NOTCH3, NOX5, NPPB, NROB1, NROB2, NRID1, NR1D2, NR1H2, NR1H3, NR1H4, NR112, NR113, NR2C1, NR2C2, NR2E1, NR2E3, NR2F1, NR2F2, NR2F6, NR3C1, NR3C2, NR4A1, NR4A2, NR4A3, NR5A1, NR5A2, NR6A1, NRP1, NRP2, NT5E, NTN4, NY-ESO1, ODZI, OPRDI, P2RX7, PAP, PART1, PATE, PAWR, P-cadherin, PCA3, PCD1, PD-L1, PCDGF, PCNA, PDGFA, PDGFB, PDGFRA, PDGFRB, PECAMI, L1-CAM, peg-asparaginase, PF4 (CXCL4), PGF, PGR, phosphacan, PIAS2, PI3 kinase, PIK3CG, PLAU (uPA), PLG, PLXDCI, PKC, PKC-beta, PPBP (CXCL7), PPID, PR1, PRAME, PRKCQ, PRKD1, PRL, PROC, PROK2, PSAP, PSCA, PSMA, PTAFR, PTEN, PTHR2, PTGS2 (COX-2), PTN, PVRIG, RAC2 (P21Rac2), RANK, RANK ligand, RARB, RGS1, RGS13, RGS3, RNFI1O (ZNF144), Ron, ROBO2, ROR1, RXR, S100A2, SCGB 1D2 (Lipophilin B), SCGB2A1 (mammaglobin 2), SCGB2A2 (mammaglobin 1), SCYE1 (endothelial monocyte-activating cytokine), SDF2, SERPENA1, SERPINA3, SERPINB5 (maspin), SERPINEI (PAI-I), SERPINFI, SHIP-1, SHIP-2, SHB1, SHB2, SHBG, SfcAZ, SLAMF7, SLC2A2, SLC33A1, SLC43A1, SLC44A4, SLC34A2, SLIT2, SPP1, SPRR1B (Spr1), ST6GAL1, ST8SIA1, STAB1, STATE, STEAP, STEAP2, TB4R2, TBX21, TCP1O, TDGF1, TEK, TGFA, TGFB1, TGFB1I1, TGFB2, TGFB3, TGFBI, TGFBR1, TGFBR2, TGFBR3, THIL, THBS1 (thrombospondin-1), THBS2, THBS4, THPO, TIE (Tie-1), TIMP3, tissue factor, TLR1, TLR2, TLR3, TLR4, TLR5, TLR6, TLR7, TLR8, TLR9, TLR10, TLR11, TNF, TNF-a, TNFAIP2 (B94), TNFAIP3, TNFRSFI1A, TNFRSF1A, TNFRSF1B, TNFRSF21, TNFRSF5, TNFRSF6 (Fas), TNFRSF7, TNFRSF8, TNFRSF9, TNFSF1O (TRAIL), TNFRSF10A, TNFRSF10B, TNFRSF12A, TNFRSF17, TNFSF1 1 (TRANCE), TNFSF12 (APO3L), TNFSF13 (April), TNFSF13B, TNFSF14 (HVEML), TNFRSF14 (HVEM), TNFSF15 (VEGI), TNFSF18, TNFSF4 (OX40 ligand), TNFSF5 (CD40 ligand), TNFSF6 (FasL), TNFSF7 (CD27 ligand), TNFSF8 (CD30 ligand), TNFSF9 (4-1BB ligand), TOLLIP, Toll-like receptor, TOP2A (topoisomerase Iia), TP53, TPM1, TPM2, TRADD, TRAF1, TRAF2, TRAF3, TRAF4, TRAF5, TRAF6, TRKA, TREM1, TREM2, TROP2, TRPC6, TSLP, TWEAK, Tyrosinase, uPAR, VEGF, VEGFB, VEGFC, versican, VHL C5, VLA-4, WT1, Wnt-1, XCL1 (lymphotactin), XCL2 (SCM-Ib), XCRI (GPR5/CCXCR1), YY1, ZFPM2, CLEC4C (BDCA-2, DLEC, CD303, CDH6, CLECSF7), CLEC4D (MCL, CLECSF8), CLEC4E (Mincle), CLEC6A (Dectin- 2), CLEC5A (MDL-1, CLECSF5), CLEC1B (CLEC-2), CLEC9A (DNGR-1), CLEC7A (Dectin- 1), CLEC11A, PDGFRa, SLAMF7, GP6 (GPVI), LILRA1 (CD85I), LILRA2 (CD85H, ILT1), LILRA4 (CD85G, ILT7), LILRA5 (CD85F, ILT11), LILRA6 (CD85b, ILT8), LILRB1, NCR1 (CD335, LY94, NKp46), NCR3 (CD335, LY94, NKp46), NCR3 (CD337, NKp30), OSCAR, TARM1, CD30, CD300C, CD300E, CD300LB (CD300B), CD300LD (CD300D), KIR2DL4 (CD158D), KIR2DS, KLRC2 (CD159C, NKG2C), KLRK1 (CD314, NKG2D), NCR2 (CD336, NKp44), PILRB, SIGLEC1 (CD169, SN), SIGLEC5, SIGLEC6, SIGLEC7, SIGLEC8, SIGLEC9, SIGLEC10, SIGLEC11, SIGLEC12, SIGLEC14, SIGLEC15 (CD33L3), SIGLEC16, SIRPA, SIRPB1 (CD172B), TREM1 (CD354), TREM2, KLRF1 (NKp80), 17-1A, SLAM7, MSLN, CTAG1B/NY-ESO-1, MAGEA3/A6, ATP5I (Q06185), OAT (P29758), AIFM1 (Q9Z0X1), AOFA (Q64133), MTDC (P18155), CMC1 (Q8BH59), PREP (Q8K411), YMEL1 (O88967), LPPRC (Q6PB66), LONM (Q8CGK3), ACON (Q99KI0), ODO1 (Q60597), IDHP (P54071), ALDH2 (P47738), ATPB (P56480), AATM (P05202), TMM93 (Q9CQW0), ERGI3 (Q9CQE7), RTN4 (Q99P72), CL041 (Q8BQR4), ERLN2 (Q8BFZ9), TERA (Q01853), DAD1 (P61804), CALX (P35564), CALU (O35887), VAPA (Q9WV55), MOGS (Q80UM7), GANAB (Q8BHN3), ERO1A (Q8R180), UGGG1 (Q6P5E4), P4HA1 (Q60715), HYEP (Q9D379), CALR (P14211), AT2A2 (055143), PDIA4 (P08003), PDIA1 (P09103), PDIA3 (P27773), PDIA6 (Q922R8), CLH (Q68FD5), PPIB (P24369), TCPG (P80318), MOT4 (P57787), NICA (P57716), BASI (P18572), VAPA (Q9WV55), ENV2 (P11370), VAT1 (Q62465), 4F2 (P10852), ENOA (P17182), ILK (O55222), GPNMB (Q99P91), ENV1 (P10404), ERO1A (Q8R180), CLH (Q68FD5), DSG1A (Q61495), AT1A1 (Q8VDN2), HYOU1 (Q9JKR6), TRAP1 (Q9CQN1), GRP75 (P38647), ENPL (P08113), CH60 (P63038), or CH10 (Q64433), but not limited thereto.

The target antigen may be an antigen that is expressed at least 10-fold more in cancer cells compared to normal cells.

### [Pharmaceutically Acceptable Salts]

In the specification, the term "pharmaceutically acceptable salt" refers to salts commonly used in the pharmaceutical field, including, but not limited to, salts of inorganic cations such as sodium, potassium, calcium, magnesium, lithium, copper, manganese, zinc, and iron; salts of inorganic acids such as hydrochloric acid, phosphoric acid, and sulfuric acid; and salts of organic acids such as ascorbic acid, citric acid, tartaric acid, lactic acid, maleic acid, malonic acid, fumaric acid, glycolic acid, succinic acid, propionic acid, acetic acid, orotic acid, and acetylsalicylic acid; and amino acid salts such as lysine, arginine, and guanidine. In addition, pharmaceutically acceptable salts may include organic ionic salts such as tetramethylammonium, tetraethylammonium, tetrapropylammonium, tetrabutylammonium, benzyltrimethylammonium, and benzethonium, which may be used in pharmaceutical reactions, purification, and separation processes. However, the types of salts referred to in the present invention are not limited to those listed above.

### [Carrier-Drug Conjugates]

According to the present invention, the two selected drug-linker conjugates may be applied to various types of drug carriers (other than antibodies), which exhibit superior tumor penetration and favorable for CMC, and are therefore highly versatile for various applications.

The carrier may be an antigen-binding fragment of an antibody, a repebody, and/or an aptamer.

An aptamer is a DNA or RNA-based biopolymer that, like antibodies, has nanomolar-level binding affinity for antigens and can be used as a cancer tissue-selective carrier for drug delivery.

A repebody, a novel class of immune protein derived from fish, can recognize antigens similarly to antibodies and nanobodies (e.g., single-chain antibodies derived from llamas or other mammals). It also exhibits an antigen binding affinity in the nanomolar (nM) range with respect to the antigen, and thus can be used as a cancer tissue-selective carrier for drugs. In particular, due to its non-immunogenicity, it can serve as a drug carrier, and its low molecular weight allows for enhanced tumor cell penetration. Furthermore, it is suitable for the production of drug conjugates with improved tumor tissue penetration, as it enables continuous production of repebodies with consistent quality.

### [pharmaceutical composition for preventing or treating cancer]

The present invention provides a pharmaceutical composition for preventing or treating cancer, comprising the antibody-drug conjugate (ADC) according to the present invention or a pharmaceutically acceptable salt thereof as an active ingredient.

In one embodiment, the present invention provides a method for preventing or treating cancer, comprising administering a therapeutically effective amount of the ADC to a subject in need thereof. As used herein, the term "subject" refers to any animal, including humans, livestock, and rodents, that either has developed or is at risk of developing the disease to be treated, and may exclude humans in certain embodiments.

Because the ADC of the present invention specifically binds to an antigen expressed on cancer cells and releases the cytotoxic drug inside or outside the cancer cell to exert cytotoxicity, it may be usefully employed in the prevention or treatment of cancer. The anticancer activity of the immunoconjugate according to the present invention is as described above.

In the present invention, the cancer may be a solid cancer or a hematological cancer. For example, the cancer may be one or more selected from the group consisting of pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungiodes, acute myeloid leukemia, acute lymphoblastic leukemia, basal cell carcinoma, ovarian epithelial carcinoma, ovarian germ cell carcinoma, male breast cancer, brain cancer, pituitary adenoma, multiple myeloma, gallbladder cancer, bile duct cancer, colorectal cancer, chronic myelogenous leukemia, chronic lymphocytic leukemia, retinoblastoma, choroidal melanoma, ampulla of Vater cancer, bladder cancer, peritoneal cancer, parathyroid cancer, adrenal cancer, paranasal sinus cancer, non-small cell lung cancer, tongue cancer, astrocytoma, small cell lung cancer, pediatric brain cancer, pediatric lymphoma, childhood leukemia, small bowel cancer, meningioma, esophageal cancer, glioma, renal pelvis cancer, kidney cancer, heart cancer, duodenal cancer, malignant soft tissue cancer, malignant bone cancer, malignant lymphoma, malignant mesothelioma, malignant melanoma, eye cancer, vulvar cancer, ureteral cancer, urethral cancer, unknown primary neoplasm, gastric lymphoma, gastric cancer, gastric carcinoma, gastrointestinal stromal cancer, Wilms' cancer, breast cancer, sarcoma, penile cancer, pharyngeal cancer, gestational trophoblastic disease, cervical cancer, endometrial cancer, uterine sarcoma, prostate cancer, metastatic bone cancer, metastatic brain cancer, mediastinal cancer, rectal cancer, occipital carcinoid tumors, vaginal cancer, spinal cancer, vestibular schwannoma, pancreatic cancer, salivary gland cancer, Kaposi's sarcoma, Paget's disease, tonsil cancer, squamous cell carcinoma, lung adenocarcinoma, lung cancer, squamous cell lung cancer, skin cancer, anal cancer, rhabdomyosarcoma, laryngeal cancer, pleural cancer, blood cancer, and thymic carcinoma, but not limited thereto. In addition the cancer includes not only primary cancer but also metastatic cancer.

As used in the present invention, the term "therapeutically effective amount" refers to an amount of the immunoconjugate effective for the prevention or treatment of cancer. Specifically, a "therapeutically effective amount" refers to an amount sufficient to treat a disease with a reasonable benefit/risk ratio applicable to medical treatment. The effective dosage level may be determined according to factors well known in the medical field, including type of subject, severity of the disease, age, sex, type of the disease, activity of drug, sensitivity to the drug, administration timing, administration route, rate of excretion, treatment period, concomitant drugs, and other relevant factors. The pharmaceutical composition of the present invention may be administered alone or in combination with other therapeutic agents, and may be administered sequentially or simultaneously with a marketed therapeutic product. It may be administered as a single or multiple dose. It is important to administer the minimum amount that produces the maximum therapeutic effect without side effects, taking into account all such factors. Since the immunoconjugate of the present invention exhibits a dose-dependent effect, the administration dose can be easily determined by those skilled in the art based on the patient's condition, age, sex, and complications. The active ingredient of the pharmaceutical composition of the present invention is highly safe and thus may be used even above the determined dosage.

Furthermore, in one embodiment of the present invention, there is provided the use of the immunoconjugate in the preparation of a medicament for preventing or treating cancer. The immunoconjugate for the preparation of a medicament may be mixed with acceptable excipients, diluents, and carriers, and may be prepared as a combination formulation with other active agents to exhibit synergistic effects of the active components.

The uses, compositions, and treatment methods described in the present invention shall be interpreted to apply equally unless otherwise inconsistent.

### ADVANTAGEOUS EFFECTS

According to the present invention, an antibody-drug conjugate (ADC) in which one or more drug-linker conjugates are conjugated to a single antibody via linkers can be used to control the pharmacokinetic properties and in vivo distribution as desired. Accordingly, for example, it is possible not only to increase the residence time in the bloodstream and maintain a high drug concentration in blood for an extended period, but also to provide the antibody-drug conjugate (ADC) with a desired hepatic clearance profile and blood circulation profile.

### BRIEF DESCRIPTION OF THE DRAWINGS

FIG. 1 is a size exclusion chromatography (SEC) analysis result of a dual-payload ADC obtained by a simultaneous reaction according to one embodiment of the present invention.
FIG. 2 is a hydrophobic interaction chromatography (HIC) analysis result of a dual-payload ADC prepared by a simultaneous reaction according to one embodiment of the present invention.
FIG. 3 is an SDS-PAGE analysis result of a dual-payload ADC prepared by a simultaneous reaction according to one embodiment of the present invention.
FIG. 4 shows the correlation between the mixing ratio and DAR (drug-to-antibody ratio) of the dual-payload ADC prepared by a simultaneous reaction according to one embodiment of the present invention.
FIG. 5 is a result of analyzing cell viability following treatment with the dual-payload ADC prepared according to one embodiment of the present invention.
FIG. 6 is a result of analyzing cell viability following treatment with the dual-payload ADC prepared according to one embodiment of the present invention.

### BEST MODE FOR CARRYING OUT THE INVENTION

The present invention will now be described in further detail with reference to the following Examples. However, the following Examples are provided merely to illustrate the technical features of the present invention more clearly and are not intended to limit the scope of the present invention.

### [Example]

### Example 1: Preparation of Comparative Single Payload ADC

The antibody was buffer-exchanged using a PD-10 desalting column into a reduction buffer (150 mM NaCl, 50 mM Histidine, pH 6.0). To a 4 mg/mL solution of the antibody, 9 molar equivalents of TCEP were added and the mixture was incubated at 25°C for 2 hours to reduce the disulfide bonds of the antibody.

The reduced antibody was subjected to removal of excess TCEP using a PD-10 desalting column, and then a conjugation reaction was carried out by reacting the reduced antibody (2 mg/mL) with 12 molar equivalents of linker-payload 25-6 (as represented by Chemical Formula 3) in a reaction buffer containing 10% DMSO (25 mM Histidine, pH 6.0) at 25°C for 1 hour.

After the conjugation reaction, the excess linker-payload mixture was removed using a PD-10 desalting column to obtain final ADC 0 (DAR 8; ADC conjugated with 25-6 at DAR 8).

### Example 2: Method for Preparing Dual-Payload ADCs Containing Different Payload 1 and 2

### 2-1. Preparation of Dual-Payload ADCs by Simultaneous Reaction of Two Linker-Payloads

The antibody was buffer-exchanged using a PD-10 desalting column into a reduction buffer (150 mM NaCl, 50 mM Histidine, pH 6.0). A 4 mg/mL solution of the antibody was treated with 9 molar equivalents of TCEP at 25°C for 2 hours to reduce its disulfide bonds.

Three mixtures of linker-payloads were prepared using mc-val-cit-PAB-MMAE (Maleimide-Caproyl amide-Val-Cit-PAB-MMAE; hereinafter referred to as vcMMME) dissolved in DMSO and linker-payload 25-6 (represented by Chemical Formula 3), in molar ratios of 2:6, 3:5, and 4:4.

After removing excess TCEP via PD-10 desalting column, conjugation reactions were performed by mixing 2 mg/mL of the reduced antibody with 12 molar equivalents of each linker-payload mixture in reaction buffer (25 mM Histidine, pH 6.0, with 10% DMSO) at 25°C for 1 hours.

The excess linker-payload mixtures were removed via PD-10 desalting column, and final dual-payload ADCs 1-7 were obtained.

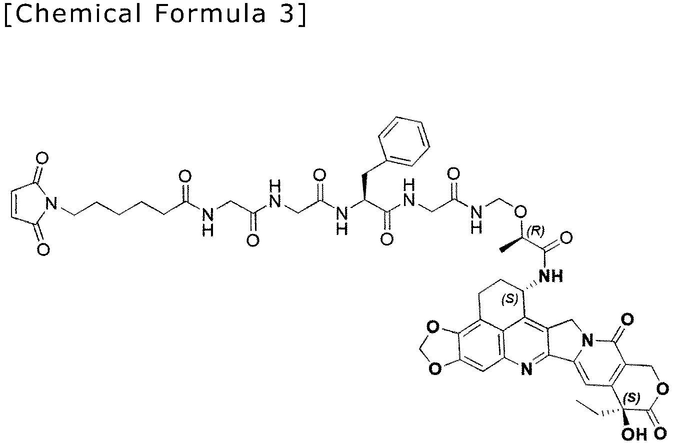

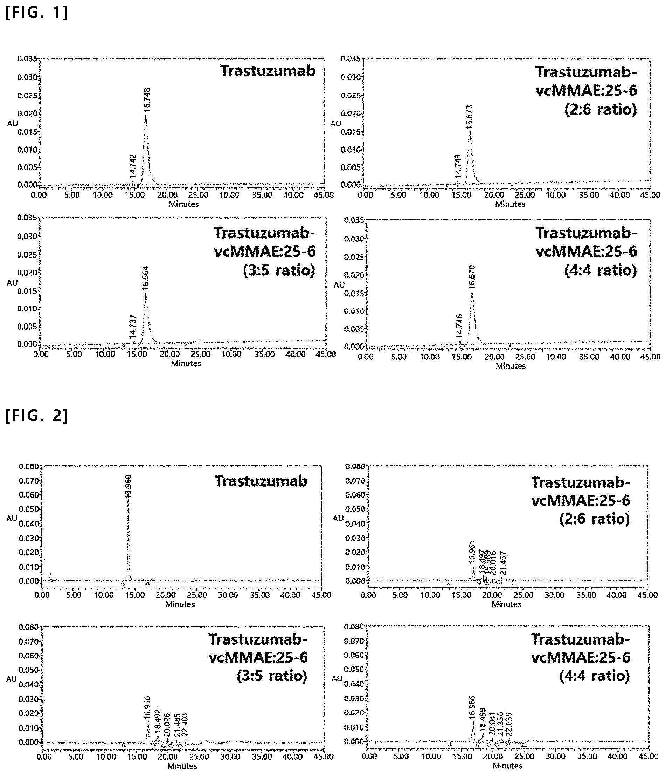

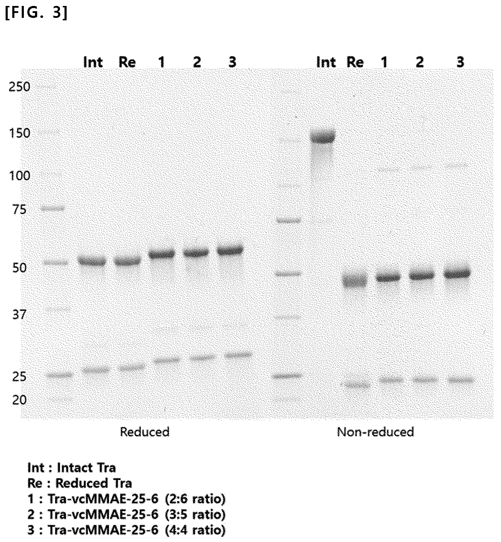

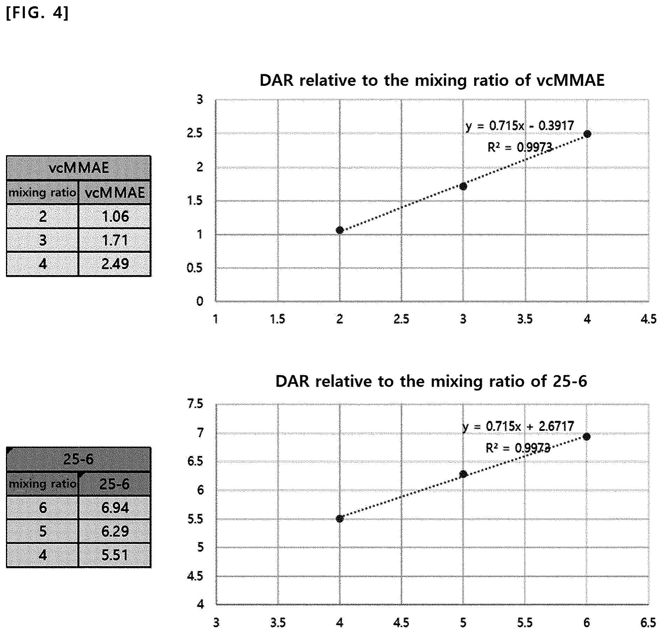

The size exclusion chromatography (SEC) analysis result of the dual-payload ADC prepared according to the present Example is shown in FIG. 1, the hydrophobic interaction chromatography (HIC) analysis result is shown in FIG. 2, the SDS-PAGE analysis result of the dual-payload ADC is shown in FIG. 3, the DAR (Drug-to-Antibody Ratio) analysis result is presented in Table 4 below, and the correlation between the reaction ratio of the dual-payload ADC and the DAR is shown in FIG. 4.

**[Table 4]**

| DAR Analysis of Dual-Payload ADC Obtained by Simultaneous Reaction | | | | |
|---|---|---|---|---|
| ADC name | Mixing ratio | | DAR | |
| | vcMMAE | 25-6 | vcMMAE | 25-6 |
| ADC 1 | 2 | 6 | 1.06 | 6.94 |
| ADC 2 | 3 | 5 | 1.71 | 6.29 |
| ADC 3 | 4 | 4 | 2.49 | 5.51 |

**[Table 5]**

| Verification of Reproducibility in the Preparation of Dual-Payload ADCs via Simultaneous Reaction | | | | |
|---|---|---|---|---|
| ADC name | Mixing ratio | | DAR | |
| | vcMMAE | 25-6 | vcMMAE | 25-6 |
| ADC 4 | 2 | 6 | 1.15 | 6.85 |
| ADC 5 | | | 0.96 | 7.04 |
| ADC 6 | 4 | 4 | 2.44 | 5.56 |
| ADC 7 | | | 2.52 | 5.48 |

### 2-2. Preparation Method of Dual-Payload ADC via Sequential Reaction of Two Linker-Payloads

The antibody was buffer-exchanged to a reduction buffer (150 mM NaCl, 50 mM Histidine, pH 6.0) using a PD-10 desalting column. The antibody solution (4 mg/mL) was then treated with 9 molar equivalents of TCEP at 25°C for 2 hours to reduce the disulfide bonds of the antibody.

The reduced antibody was purified using a PD-10 desalting column to remove excess TCEP. In a reaction buffer (25 mM Histidine, pH 6.0) containing 10% DMSO, the reduced antibody (2 mg/mL) was reacted with 2.0, 2.1, 2.2 or 4.0, 4.1, 4.2 molar equivalents of mc-vc-MMAE linker-payload at 25°C for 1 hour to perform the first conjugation reaction.

To the first reaction mixture, 12 molar equivalents of the 25-6 linker-payload were added, and the mixture was reacted at 25°C for 1 hour to perform the second conjugation reaction.

After all reactions, excess linker-payloads were removed using a PD-10 desalting column, and the final dual-payload ADCs (ADC 8-13) were obtained.

**[Table 6]**

| SDS-PAGE Analysis of Dual-Payload ADCs Obtained via Sequential Reaction | | | | |
|---|---|---|---|---|
| ADC name | stoichiometric equivalent | | DAR | |
| | vcMMAE | 25-6 | vcMMAE | 25-6 |
| ADC 8 | 2.0 | 12 | 1.96 | 6.04 |
| ADC 9 | 2.1 | | 1.99 | 6.01 |
| ADC 10 | 2.2 | | 2.13 | 5.87 |
| ADC 11 | 4.0 | 12 | 3.81 | 4.19 |
| ADC 12 | 4.1 | | 3.97 | 4.03 |
| ADC 13 | 4.2 | | 4.01 | 3.99 |

### 2-3. Cell viability assay

For various cancer cell lines, 3,000 cells per well (KPL4, FaDu, BT474, MDA-MB-468) were seeded into 96-well plates and incubated under standard conditions (37°C, 5% CO2). After 24 hours, ADC samples (100 µL) at 9 different concentrations (serially diluted 1:5 from 1000 nM) were added to the cells. At this time, a single payload ADC, Tra-25-6 (DAR8), and a dual payload ADC, Tra-vcMMAE-25-6 (DAR1+7), were treated, and a control group (ADC concentration 0) without any ADC sample was also prepared. After incubation under standard conditions (37°C, 5% CO2) for 6 days, 100 µL of CellTiter-Glo reagent (CellTiter-Glo^{®} Luminescent Cell Viability Assay kit(Promega, G7571)) was added to each well, pipetted, incubated at room temperature (RT) for 10 minutes, and then luminescence was measured. When the luminescence value at ADC concentration 0 is regarded as 100%, the concentration showing 50% luminescence is defined as the IC₅₀ value.

### 2-4. Cell Viability Assay Results

To evaluate the in vitro efficacy of the dual payload ADC compared to the single payload ADC depending on the expression level of HER2, single payload ADC (Tra-25-6 (DAR8)) and dual payload ADC (Tra-vcMMAE-25-6 (DAR1+7)) were each administered to various cancer cell lines exhibiting different levels of HER2 expression, and cell viability was monitored after 6 days of incubation.

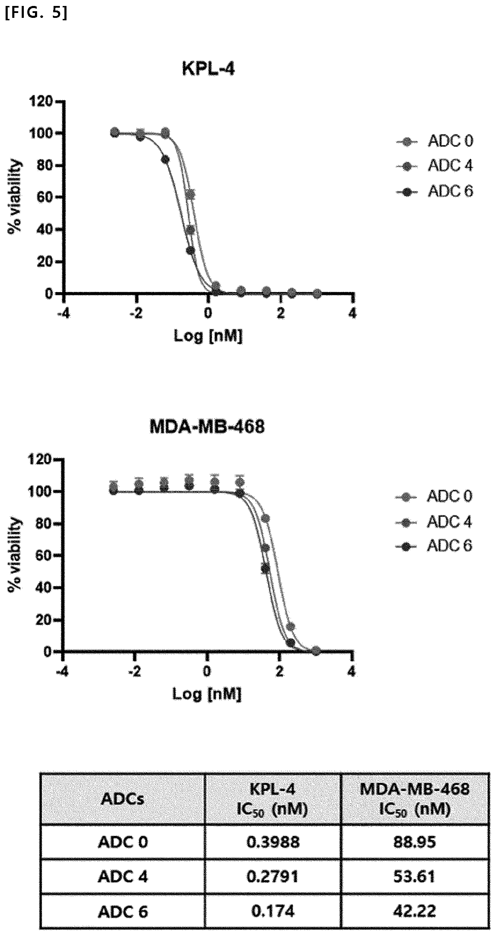

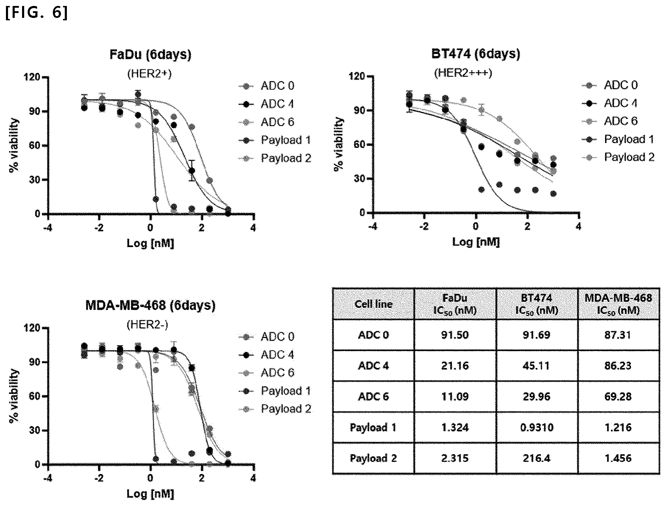

Referring to FiGS 5 and 6, it was confirmed that the dual payload ADCs, ADC 4 and ADC 6, exhibited lower IC₅₀ values and stronger cytotoxic efficacy compared to the single payload ADC (ADC 0; Tra-25-6 (DAR(0+8))) in HER2-positive cell lines. Furthermore, depending on the level of HER2 expression, it was confirmed that the dual payload ADCs, ADC 4 and ADC 6, exhibited lower IC₅₀ values compared to the single payload ADC (ADC 0) not only in cell lines with high HER2 expression (KPL4, BT474) but also in cell lines with relatively moderate expression (FaDu), thereby demonstrating that the cell-killing effect of the dual payload ADCs is stronger than that of the single payload ADC.

This confirms that two payloads with different mechanisms of action (topoisomerase I inhibitor and microtubule inhibitor) can synergistically contribute to enhanced efficacy.

In contrast, in HER2-negative cell lines, both Tra-25-6 (DAR8) and Tra-vcMMAE-25-6 (DAR1+7) exhibited similar ICso values, indicating that no additional cell-killing effect was induced and that both ADCs showed comparable cytotoxicity.

In addition, the difference in cytotoxicity depending on the sensitivity of each cancer cell line to the respective drug payloads was observed.

That is, according to present Example, the antibody-drug conjugate (ADC) may include one or more drug-linker conjugates, and when the drugs included in each drug-linker conjugate are of different types, a significant synergistic effect on cancer cell killing is exhibited.

The present invention has thus far been described with reference to its preferred embodiments.

In the Example, it was demonstrated that in the preparation of a dual payload ADC with DAR 8 composed of two different payloads 1 and 2, the respective DARs of payloads 1 and 2 can be freely adjusted to 1 and 7, 2 and 6, 3 and 5, or 4 and 4. This is achievable through simultaneous or sequential reaction by adjusting the molar ratios of the reactants. The method of the present invention also allows for the preparation of dual payload ADCs with DAR 4 or DAR 6. For example, in the case of DAR 4, the DARs of each payload can be adjusted to 1 and 3 or 2 and 2; in the case of DAR 6, the DARs can be adjusted to 1 and 5, 2 and 4, or 3 and 3. Those skilled in the art will readily understand that the present invention can be implemented in a modified form without departing from its essential characteristics. Therefore, the disclosed Examples should be considered illustrative rather than limiting. The scope of the present invention is defined by the claims, and all modifications and variations equivalent thereto are to be construed as being included within the scope of the present invention.

## Claims

1. An antibody-drug conjugate (ADC), in which one or more drug-linker conjugates are conjugated to an antibody,
wherein the one or more drug-linker conjugates are selected from the group consisting of:
a drug-linker conjugate(A) consisting of the combination of a camptothecin-based drug and an acid-sensitive linker;
a drug-linker conjugate(B) consisting of the combination of a non-camptothecin-based super-toxic drug and an enzyme-sensitive linker;
a drug-linker conjugate(C) consisting of the combination of a camptothecin-based drug and an enzyme-sensitive linker; and
a drug-linker conjugate(D) consisting of the combination of an anti-apoptotic protein inhibitor drug and an enzyme-sensitive linker,
and wherein each of the one or more drug-linker conjugates is conjugated to an amino acid residue comprising cysteine or lysine, a non-natural amino acid residue, or a glycan of the antibody.

2. An antibody-drug conjugate (ADC), in which a first drug-linker conjugate and a second drug-linker conjugate are conjugated to a single antibody,
wherein the first drug-linker conjugate(B) consists of the combination of a non-camptothecin supertoxin drug and an enzyme-sensitive linker;
and wherein the second drug-linker conjugate(C) consists of a combination of a camptothecin-based drug and an enzyme-sensitive linker.

3. The antibody-drug conjugate of claim 2,
wherein the drug-to-antibody ratio(DAR) of the antibody-drug conjugate is from 4 to 8.

4. The antibody-drug conjugate of claim 2,
wherein the first drug-linker conjugate is a compound of Chemical Formula 6 or 7 below, and wherein the second drug-linker conjugate is a compound selected from Chemical Formula 2 to 5 below;

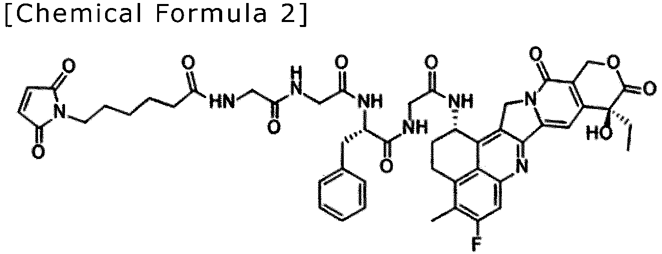

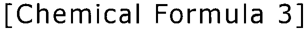

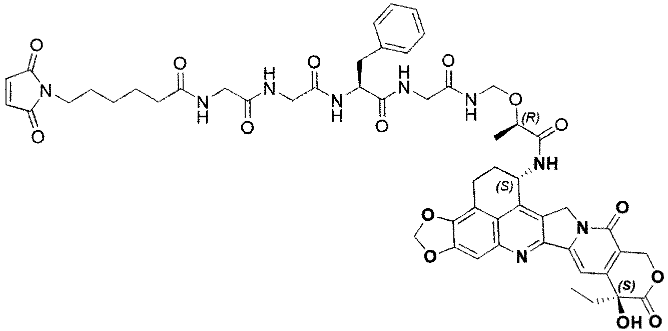

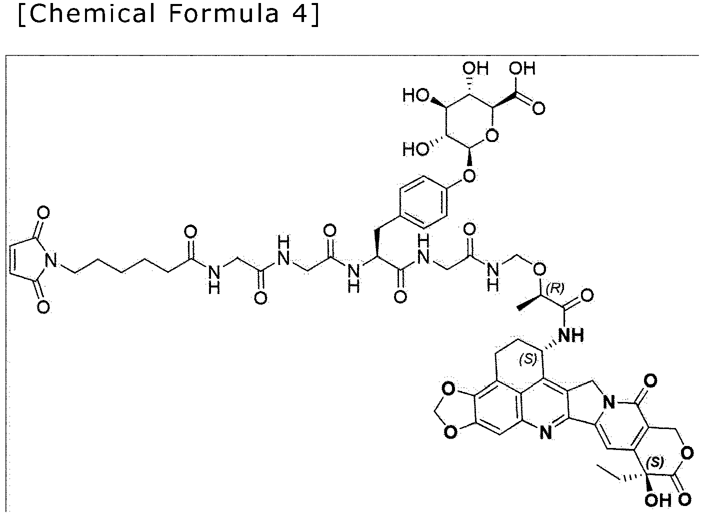

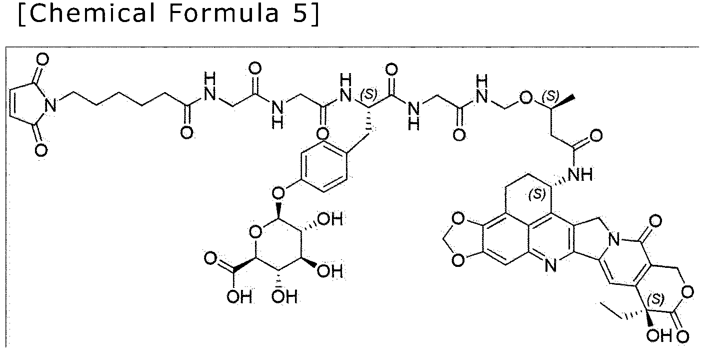

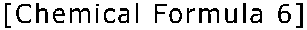

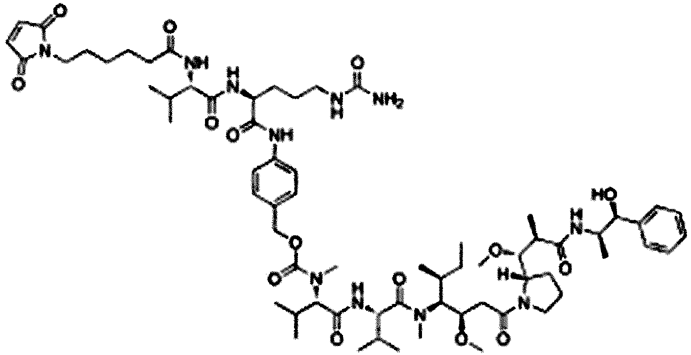

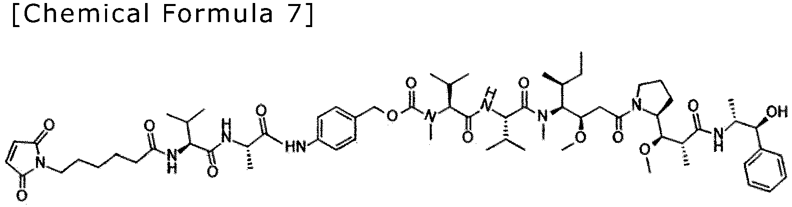

5. The antibody-drug conjugate of claim 2,
wherein a drug included in the first drug-linker conjugate and drug included in the second drug-linker conjugate are conjugated to the antibody in a ratio of 1:1 to 1:7.

6. The antibody-drug conjugate of claim 2,
wherein the antibody-drug conjugate is prepared by a sequential reaction or a simultaneous reaction.

7. The antibody-drug conjugate of claim 2,
wherein a drug included in the first drug-linker conjugate is an Auristatin-based compound,
and wherein a drug included in the second drug-linker conjugate is a camptothecin-based derivative that inhibits Topoisomerase I.

8. The antibody-drug conjugate of claim 7,
wherein the drug included in the first drug-linker conjugate is an Auristatin-based compound selected from the group consisting of MMAE, MMAU, Aur0101, Duostatin 5 and Microtubule inhibitor,
and wherein the drug included in the second drug-linker conjugate is a topoisomerase I inhibitor, the topoisomerase I inhibitor is at least one compound selected from the group consisting of SN-38, Exatecan, Dxd, FL118 and 7-aminoalkylsubstituted camptothecin compound, or a derivative thereof.

9. The antibody-drug conjugate of claim 2,
wherein the antibody is at least one selected from the group consisting of Urelumab, Utomilumab, Bebtelovimab, Aducanumab, Bapinezumab, Crenezumab, Donanemab, Gantenerumab, Lecanemab, Solanezumab, Nesvacumab, Evinacumab, Enoblituzumab, Omburtamab, Belimumab, Ianalumab, Tabalumab, Bertilimumab, Mogamulizumab, Leronlimab, Siplizumab, Foralumab, Muromonab-CD3, Otelixizumab, Teplizumab, Ibalizumab, Tregalizumab, Zanolimumab, Itolizumab, Efalizumab, Inebilizumab, Tafasitamab, Tositumomab, Ocrelizumab, Ofatumumab, Rituximab, Ublituximab, Veltuzumab, Epratuzumab, Basiliximab, Daclizumab, Varlilumab, Lulizumab, Iratumumab, Lintuzumab, Daratumumab, Felzartamab, Isatuximab, Mezagitamab, Bleselumab, Dacetuzumab, Iscalimab, Lucatumumab, Mitazalimab, Sotigalimab, Dapirolizumab, Apamistamab, Ligufalimab, Magrolimab, Alemtuzumab, Crizanlizumab, Inclacumab, Cusatuzumab, Oleclumab, Milatuzumab, Galiximab, Carotuximab, Adecatumumab, Eptinezumab, Erenumab, Fremanezumab, Galcanezumab, Zolbetuximab, Onartuzumab, Eculizumab, Pozelimab, Ravulizumab, Lacnotuzumab, Axatilimab, Cabiralizumab, Emactuzumab, Ipilimumab, Quavonlimab, Tremelimumab, Zalifrelimab, Cetuximab, Depatuxizumab, Futuximab, Imgatuzumab, Matuzumab, Modotuximab, Necitumumab, Nimotuzumab, Panitumumab, Tomuzotuximab, Zalutumumab, Batoclimab, Nipocalimab, Rozanolixizumab, Burosumab, Farletuzumab, Dinutuximab, Naxitamab, Ragifilimab, Gimsilumab, Lenzilumab, Mavrilimumab, Namilumab, Otilimab, Plonmarlimab, Codrituzumab, Margetuximab, Pertuzumab, Trastuzumab, Datopotamab, Patritumab, Seribantumab, Duligotuzumab, Ficlatuzumab, Rilotumumab, Alomfilimab, Anifrolumab, Emapalumab, Ligelizumab, Omalizumab, Cixutumumab, Dalotuzumab, Figitumumab, Ganitumab, Teprotumumab, Bermekimab, Canakinumab, Gevokizumab, Briakinumab, Ustekinumab, Anrukinzumab, Cendakimab, Lebrikizumab, Tralokinumab, Brodalumab, Bimekizumab, Ixekizumab, Secukinumab, Brazikumab, Guselkumab, Mirikizumab, Risankizumab, Tildrakizumab, Nemolizumab, Imsidolimab, Spesolimab, Pascolizumab, Dupilumab, Depemokimab, Mepolizumab, Reslizumab, Benralizumab, Clazakizumab, Olokizumab, Siltuximab, Sirukumab, Ziltivekimab, Levilimab, Sarilumab, Satralizumab, Tocilizumab, Abituzumab, Favezelimab, Fianlimab, Ieramilimab, Relatlimab, Simtuzumab, Abagovomab, Oregovomab, Tanezumab, Ivuxolimab, Rocatinlimab, Tavolimab, Telazorlimab, Vonlerolizumab, Alirocumab, Bococizumab, Ebronucimab, Evolocumab, Frovocimab, Ongericimab, Tafolecimab, Dostarlimab, Balstilimab, Camrelizumab, Cemiplimab, Geptanolimab, Nivolumab, Pembrolizumab, Penpulimab, Pidilizumab, Prolgolimab, Retifanlimab, Sasanlimab, Serplulimab, Sintilimab, Spartalizumab, Tislelizumab, Toripalimab, Ezabenlimab, Zimberelimab, Atezolizumab, Avelumab, Cosibelimab, Sugemalimab, Durvalumab, Envafolimab, Suvratoxumab, Denosumab, Zilovertamab, Elotuzumab, Domvanalimab, Etigilimab, Ociperlimab, Tiragolumab, Vibostolimab, Surzebiclimab, Cobolimab, Sabatolimab, Concizumab, Marstacimab, Adalimumab, Golimumab, Infliximab, Certolizumab, Conatumumab, Tigatuzumab, Tezepelumab, Gatipotuzumab, Cabiralizumab, Bevacizumab, Brolucizumab, Ranibizumab, Olinvacimab, Icrucumab, Ramucirumab, Caplacizumab, Abrilumab, Etrolizumab, Vedolizumab, Intetumumab, Natalizumab, Obrindatamab, Elranatamab, Linvoseltamab, Teclistamab, Epcoritamab, Glofitamab, Mosunetuzumab, Odronextamab, Flotetuzumab, Vibecotamab, Catumaxomab, Cibisatamab, Talquetamab, Ubamatamab, Emfizatamab, Blinatumomab, Amivantamab, Emicizumab, Zenocutuzumab, Zanidatamab, Tibulizumab, Naptumomab, Belantamab, Pivekimab, Praluzatamab, Coltuximab, Denintuzumab, Loncastuximab, Ibritumomab, Inotuzumab, Epratuzumab, Moxetumomab, Brentuximab, Gemtuzumab, Vadastuximab, Lorvotuzumab, Polatuzumab, Tusamitamab, Telisotuzumab, Rovalpituzumab, Depatuxizumab, Farletuzumab, Mirvetuximab, Disitamab, Anetumab, Enfortumab, Sacituzumab, Vobarilizumab, Cadonilimab, Vudalimab, Tebotelimab, Ivonescimab, Erfonrilimab, Ozoralizumab, Faricimab, Vanucizumab, and Navicixizumab.

10. A method for preparing an antibody-drug conjugate(ADC) in which one or more drug-linker conjugates are conjugated to a single antibody, comprising:
selecting one or more drug-linker conjugates from the group consisting of:
a drug-linker conjugate(A) consisting of the combination of a camptothecin-based drug and an acid-sensitive linker;
a drug-linker conjugate(B) consisting of the combination of a non-camptothecin-based super-toxic drug and an enzyme-sensitive linker;
a drug-linker conjugate(C) consisting of the combination of a camptothecin-based drug and an enzyme-sensitive linker; and
a drug-linker conjugate(D) consisting of the combination of an anti-apoptotic protein inhibitor drug and an enzyme-sensitive linker; and
conjugating each drug-linker conjugate to an amino acid residue of the antibody by employing, depending on the drug-linker conjugate, different (i) amino acid residues at the binding site on the antibody, (ii) conjugation sequences, and/or (iii) conjugation methods.

11. The method of claim 10,
wherein the (B) is a compound of Chemical Formula 6 or 7 below, and wherein the (c) is a compound selected from Chemical Formula 2 to 5 below.

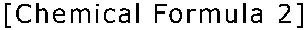

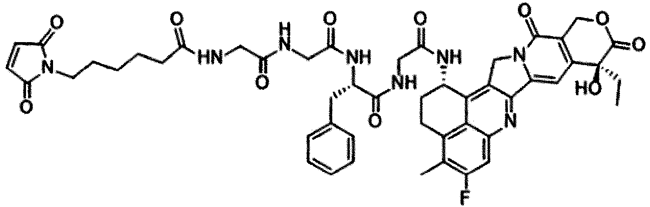

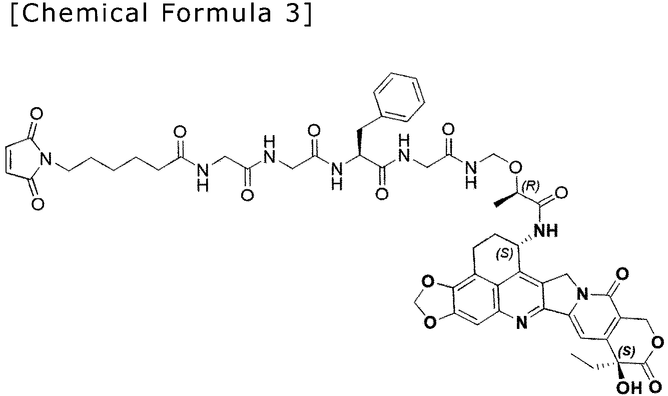

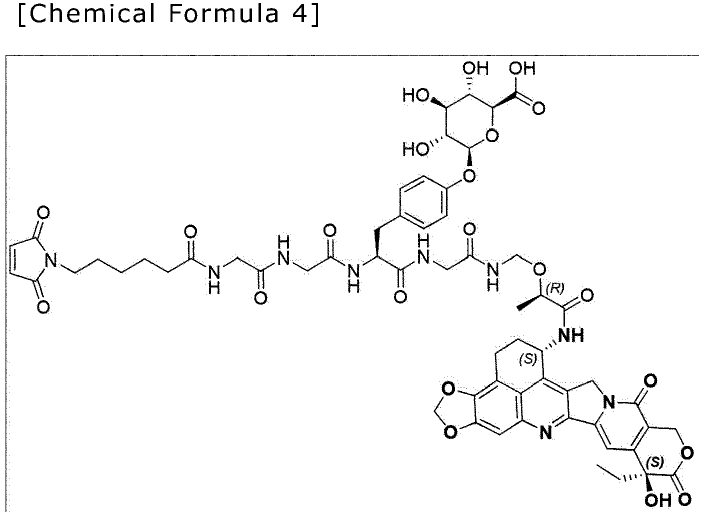

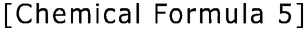

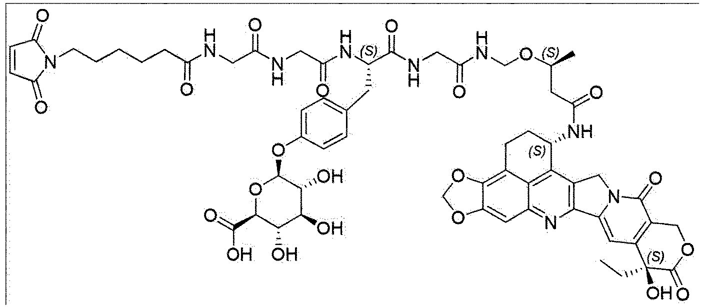

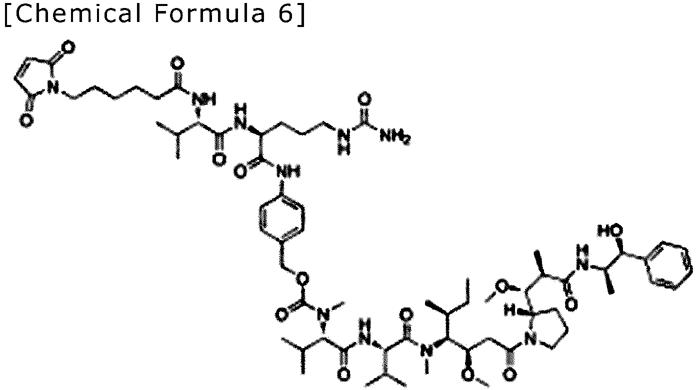

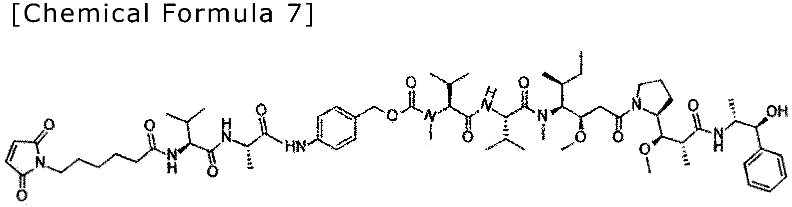

12. The method of claim 10,
wherein the conjugating, comprising preparing a mixture by mixing the drug-linker conjugates (B) and (C); and conjugating the mixture to a reduced antibody.

13. The method of claim 10,
wherein the conjugating, comprising preparing a first reaction mixture by adding the drug-linker conjugate (B) to a reduced antibody at 2- to 4-fold molar amounts relative to the antibody; preparing a second reaction mixture by adding the drug-linker conjugate (C) to the first reaction mixture.

14. A pharmaceutical composition for preventing or treating cancer, comprising an antibody-drug conjugate(ADC) in which one or more drug-linker conjugates are conjugated to an antibody,
wherein the one or more drug-linker conjugates are selected from the group consisting of:
a drug-linker conjugate(A) consisting of the combination of a camptothecin-based drug and an acid-sensitive linker;
a drug-linker conjugate(B) consisting of the combination of a non-camptothecin-based super-toxic drug and an enzyme-sensitive linker;
a drug-linker conjugate(C) consisting of the combination of a camptothecin-based drug and an enzyme-sensitive linker; and
a drug-linker conjugate(D) consisting of the combination of an anti-apoptotic protein inhibitor drug and an enzyme-sensitive linker,
and wherein the antibody-drug conjugate(ADC) is **characterized in that** each of the one or more drug-linker conjugates is conjugated to an amino acid residue comprising cysteine or lysine, a non-natural amino acid residue, or a glycan of the antibody.

15. The pharmaceutical composition of claim 14,
wherein the cancer is at least one selected from the group consisting of pseudomyxoma, intrahepatic cholangiocarcinoma, hepatoblastoma, liver cancer, thyroid cancer, colon cancer, testicular cancer, myelodysplastic syndrome, glioblastoma, oral cancer, lip cancer, mycosis fungiodes, acute myeloid leukemia, acute lymphoblastic leukemia, basal cell carcinoma, ovarian epithelial carcinoma, ovarian germ cell carcinoma, male breast cancer, brain cancer, pituitary adenoma, multiple myeloma, gallbladder cancer, bile duct cancer, colorectal cancer, chronic myelogenous leukemia, chronic lymphocytic leukemia, retinoblastoma, choroidal melanoma, ampulla of Vater cancer, bladder cancer, peritoneal cancer, parathyroid cancer, adrenal cancer, paranasal sinus cancer, non-small cell lung cancer, tongue cancer, astrocytoma, small cell lung cancer, pediatric brain cancer, pediatric lymphoma, childhood leukemia, small bowel cancer, meningioma, esophageal cancer, glioma, renal pelvis cancer, kidney cancer, heart cancer, duodenal cancer, malignant soft tissue cancer, malignant bone cancer, malignant lymphoma, malignant mesothelioma, malignant melanoma, eye cancer, vulvar cancer, ureteral cancer, urethral cancer, unknown primary neoplasm, gastric lymphoma, gastric cancer, gastric carcinoma, gastrointestinal stromal cancer, Wilms' cancer, breast cancer, sarcoma, penile cancer, pharyngeal cancer, gestational trophoblastic disease, cervical cancer, endometrial cancer, uterine sarcoma, prostate cancer, metastatic bone cancer, metastatic brain cancer, mediastinal cancer, rectal cancer, occipital carcinoid tumors, vaginal cancer, spinal cancer, vestibular schwannoma, pancreatic cancer, salivary gland cancer, Kaposi's sarcoma, Paget's disease, tonsil cancer, squamous cell carcinoma, lung adenocarcinoma, lung cancer, squamous cell lung cancer, skin cancer, anal cancer, rhabdomyosarcoma, laryngeal cancer, pleural cancer, blood cancer, and thymic carcinoma.
